# EUROPÄISCHE PATENTSCHRIFT

(11) **EP 3 765 926 B1**
(45) Veröffentlichungstag und Bekanntmachung des Hinweises auf die Patenterteilung: **13.08.2025**
(21) Anmeldenummer: 19712935.6
(22) Anmeldetag: 15.03.2019
(51) Int. Cl.: G05B 15/02, G01S 13/02

(54) **GEBÄUDE-SENSORSYSTEM**
BUILDING SENSOR SYSTEM
SYSTÈME DE CAPTEURS DE BÂTIMENT

(30) Priorität: 16.03.2018 DE 102018106145
(43) Veröffentlichungstag der Anmeldung: 20.01.2021
(73) Patentinhaber: Steinel GmbH, 33442 Herzebrock-Clarholz (DE)
(72) Erfinder: BORN, Torsten, 33813 Oerlinghausen (DE); MÖLLER, Thomas, 32049 Herford (DE)
(74) Vertreter: Mathys & Squire
(86) Internationale Anmeldenummer: PCT/EP2019/056603
(87) Internationale Veröffentlichungsnummer: WO 2019/175414

(56) Entgegenhaltungen:
- EP-A1- 1 785 029
- EP-A1- 2 784 535
- EP-A2- 0 341 022
- WO-A1-2012/110046
- DE-A1- 102014 003 253
- DE-A1- 102015 119 501
- DE-A1- 102017 100 654
- DE-U1- 202017 103 011
- US-A1- 2017 192 435

## Beschreibung

Die vorliegende Erfindung betrifft ein Gebäude-Sensorsystem nach dem Oberbegriff des Patentanspruchs 1. Außerdem betrifft die vorliegende Erfindung ein Verfahren für ein integratives Gebäudemanagement nach Anspruch 12.

Derartige Systeme und Verfahren sind im Stand der Technik bekannt und dienen beispielsweise einer situationsgerechten oder adaptiven Steuerung von Gebäudetechnik oder dergleichen.

Beispielsweise offenbart WO 2012/110046 A1 eine Vorrichtung und ein Verfahren zur Überwachung und Steuerung der Verkehrsführung an einem Flughafen, welche eine Mehrzahl von über das Flughafengelände verteilten Datenerfassungsstellen zur Erfassung von Echtzeitdaten umfasst.

Die bekannten Systeme und Verfahren haben jedoch den Nachteil, dass die Zustände der mit dem Sensorsystem überwachten oder mit dem Verfahren überwachten Raumeinheiten nur unzuverlässig oder wenig zuverlässig festgestellt werden können. Darüber hinaus haben die Verfahren und Systeme aus dem Stand der Technik den Nachteil, dass die gegebenenfalls von den Sensoren erfassten Zustände oder detektierten Ereignisse für das Gebäudemanagement zu isoliert oder zu wenig vernetzt berücksichtigt und dementsprechend das Potential der Systeme und der Verfahren nicht ausgeschöpft wird, um die Kontrolle oder das Management eines Gebäudes zu optimieren.

Ausgehend von diesem Stand der Technik liegt die Aufgabe der vorliegenden Erfindung darin ein Gebäude-Sensorsystem und ein Verfahren für ein integratives Gebäudemanagement vorzuschlagen, welches eine besonders präzise Erfassung von Zuständen einzelner Raumeinheiten ermöglicht und gleichzeitig eine möglichst weitreichende, vernetzte und damit aussagekräftige Auswertung der erfassten Zustände der Raumeinheiten ermöglicht, um somit das Gebäudemanagement zu verbessern und zu effektivieren.

Diese Aufgabe wird durch das Gebäude-Sensorsystem gemäß Anspruch 1 sowie durch das Verfahren gemäß Anspruch 12 gelöst. Vorteilhafte Weiterbildungen der Erfindung sind in den Unteransprüchen beschrieben.

Die Erfindung führt zudem zu einer Beherbergungseinrichtung, insbesondere einem Hotel, mit einem erfindungsgemäßen Sensorsystem sowie zur Verwendung des Verfahrens für das Gebäudemanagement einer Beherbergungseinrichtung, insbesondere eines Hotels. Auch führt die Erfindung zu einer Stallungseinrichtung, insbesondere zu einem Haus- und/oder Nutztierstall, besonders bevorzugt zu einem Pferdestall mit einem erfindungsgemäßen Sensorsystem sowie zu der Verwendung des erfindungsgemäßen Verfahrens für das Gebäudemanagement einer Stallungseinrichtung, insbesondere eines Haus- und/oder Nutztierstalls, besonders bevorzugt eines Pferdestalls. Ferner führt die Erfindung auch zu einer medizinischen Unterbringungseinrichtung, insbesondere Krankenhaus und/oder Alters- und/oder Pflegeheim umfassend ein erfindungsgemäßes Sensorsystem sowie zu der Verwendung des erfindungsgemäßen Verfahrens zum Gebäudemanagement einer medizinischen Unterbringungseinrichtung. Weiter wird durch die Erfindung eine Tagungs- und/oder Konferenzeinrichtung mit einem erfindungsgemäßen Sensorsystem sowie die Verwendung eines Verfahrens nach der vorliegenden Erfindung für das Gebäudemanagement einer Tages- und/oder Konferenzeinrichtung realisiert. Schließlich führt die Erfindung auch zu einer Arbeitsstätte, insbesondere Büroeinheit, mit einer Vielzahl von Arbeitsplätzen und einem erfindungsgemäßen Sensorsystem und der Verwendung des erfindungsgemäßen Verfahrens für das Gebäudemanagement einer Arbeitsstätte.

Das erfindungsgemäße Gebäude-Sensorsystem, für ein integratives Gebäudemanagement, umfasst dementsprechend eine Vielzahl von Sensormodulen, welche jeweils einer Raumeinheit zugeordnet sind und einen Zustand der Raumeinheit erfassen, wobei erfindungsgemäß vorgesehen ist, dass eine Vielzahl von Sensormodulen eine Sensoreinheit, insbesondere zur Erfassung von Mikrobewegungen, umfasst und jede Sensoreinheit mit einem Kommunikationsnetzwerk verbunden ist, über welches die Sensoreinheiten mit zumindest einer zentralen Recheneinheit kommunizieren, welche insbesondere dazu eingerichtet ist, Mikrobewegungen in den Sensorsignalen der Sensoreinheiten zu identifizieren, wobei die zentrale Recheneinheit mit zumindest einer Anwendungseinheit verbunden ist, welche dazu eingerichtet ist, Anfragen, insbesondere über Mikrobewegungen in den Raumeinheiten, an die zentrale Recheneinheit zu senden, Informationen, insbesondere über die Mikrobewegungen in den Raumeinheiten, zu empfangen, die empfangenden Informationen, insbesondere über die Mikrobewegungen in den Raumeinheiten, anwendungsbezogen und raumeinheitsbezogen zu verarbeiten und ein anwendungsspezifisches Ausgabesignal zu erzeugen.

Der Grundgedanke der Erfindung liegt folglich darin über die Recheneinheit und die Sensoreinheiten Zustände, insbesondere Mikrobewegungen, in den jeweiligen Raumeinheiten zu erfassen und zu identifizieren und aus Zuständen, insbesondere den identifizierten Mikrobewegungen anwendungsbezogen ein Ausgabesignal zu erzeugen, welches wiederum im Gebäudemanagement des Gebäudes zum Einsatz kommt, aufgegriffen wird oder weiterverwendet wird. Dadurch wird in besonders vorteilhafter Weise erreicht, dass einerseits über die Erfassung und Identifizierung von Zuständen, insbesondere Mikrobewegungen, die Zustände und die Zustandsänderungen in den Raumeinheiten besonders genau und hochauflösend erfolgen kann, und dass aufgrund der vernetzten, insbesondere raumeinheitsübergreifenden und gleichzeitig raumeinheitsbezogenen Weiterverarbeitung durch die Anwendungseinheit die erfassten und identifizierten Zustände, insbesondere Mikrobewegungen sehr effektiv und intelligent für das Gebäudemanagement genutzt und damit ein sehr genaues, effektives Gebäudemanagement erreicht werden kann. Damit wird ein Gebäudemanagement erreicht, welches den Nutzen für die Gebäudebewohner, Gebäudenutzer oder dergleichen erheblich steigert und gleichzeitig wird ein Gebäudemanagement erreicht, das besonders energieeffizient und ressourcesparend erfolgen kann.

Die Grundidee der vorliegenden Erfindung besteht weiter auch darin, dass zwar einerseits die Zustände, insbesondere Mikrobewegungen, die in den jeweiligen Raumeinheiten gemessen oder detektiert werden, einen Teil der Grundlage der Bearbeitung seitens der Anwendungseinheit ausmachen, dass aber die Bearbeitung auch anwendungsbezogene statische oder dynamische Informationen berücksichtigt, diese mit den Raumeinheiten und den dortigen Zuständen, insbesondere Mikrobewegungen verknüpft und ggf. einen größeren Kontext oder einen Gesamtkontext, der mehrere Raumeinheiten, alle Raumeinheiten oder das gesamte Gebäude mit einschließt, herstellt. So kann eine raumeinheitsbezogene Verarbeitung seitens der Anwendungseinheit zu einem Ausgabesignal führen, das Auswirkungen auf eine einzelne Raumeinheit, auf eine Vielzahl von Raumeinheiten, insbesondere auf eine Gruppe von Raumeinheiten oder auf alle Raumeinheiten bzw. das gesamte Gebäude hat. Mit anderen Worten ausgedrückt ist die Anwendungseinheit je nach Anwendung oder Teilaufgabe eine Anwendung gleichermaßen in der Lage ein Ausgabesignal zu erzeugen, welches einen klaren Bezug zu einer oder mehreren Raumeinheiten aufweist oder ein Ausgabesignal zu erzeugen, welches zwar auf Informationen bezüglich der Raumeinheiten beruht, jedoch keinen eindeutigen Bezug zu den jeweiligen Raumeinheiten aufweist.

Als Gebäude im Sinne der vorliegenden Erfindung kommt grundsätzlich jedes Bauwerk infrage. Auch bauliche Strukturen im öffentlichen Raum, die kein Gebäude im klassischen Sinne darstellen, können im Zusammenhang mit der vorliegenden Erfindung als Gebäude betrachtet werden. Die Raumeinheiten können jeweils in sich geschlossene und abgetrennte Räume darstellen. Alternativ kann jedoch das System auch bei Raumeinheiten zum Einsatz kommen, die einen einzigen Raum in mehrere, insbesondere nicht baulich getrennte Untereinheiten, also Raumeinheiten trennt. Beispielsweise kann als Raumeinheit ein einzelnes Zimmer infrage kommen. Alternativ kann eine Raumeinheit jedoch auch einen Bereich eines größeren Raumes, beispielsweise einen Bereich eines Saals, einen Abschnitt einer Tief- oder Parkgarage oder ein Gleisabschnitt eines Bahnhofs umfassen. Auch mehrere Gebäude können im Sinne der vorliegenden Erfindung als eine Raumeinheit verstanden werden.

Auf die Sensormodule und die Sensoreinheiten der Sensormodule wird in der nachfolgenden Offenbarung noch detaillierter eingegangen werden. An dieser Stelle soll lediglich erläutert werden, dass die Sensoreinheiten bevorzugt zur Erfassung von Mikrobewegungen derart eingerichtet sind, dass Bewegungen von einigen Millimetern, bevorzugt Bewegungen von lediglich einem Millimeter oder weniger erfasst und identifiziert werden können. Für die Verbindung zwischen den Sensoreinheiten und der Recheneinheit sowie für die Verbindung zwischen der Recheneinheit und der Anwendungseinheit sowie für eine weitere im Rahmen der nachfolgenden Offenbarung beschriebenen Verbindungen zwischen Einheiten des Systems kommen, wenn nicht anders spezifiziert, grundsätzlich alle jeweils geläufigen und gängigen datentechnischen Verbindungen sowie deren gegenständliche Verbindungsmittel und verfahrensmäßigen Verbindungs- oder Übertragungsprotokolle in Betracht. Beispielsweise können kabelgebundene oder drahtlose Verbindungen zum Einsatz kommen. Zumindest eine Anwendungseinheit kann gemäß der vorliegenden Erfindung als separate gegenständliche Einheit ausgeführt sein. Alternativ kann sie jedoch auch von der zentralen Recheneinheit mit umfasst sein.

Die Identifizierung von Zuständen, insbesondere Mikrobewegungen kann einerseits bereits sensorseitig erfolgen, andererseits kann die Aufgabe auch durch die zentrale Recheneinheit gewährleistet werden. Für den Fall, dass Mikrobewegungen von den Sensormodulen oder den Sensoreinheiten nicht nur erfasst, sondern auch identifiziert werden sollen, kann vorteilhaft vorgesehen sein, dass die Sensoreinheiten, insbesondere die Sensormodule eine dazu eingerichtete Sensorrecheneinheit aufweisen. Dies kann vorteilhaft die zentrale Recheneinheit entlasten.

Die zentrale Recheneinheit kann beispielsweise als Server ausgestaltet sein. Dazu kann eine dem System räumlich zugeordnete Servereinrichtung vorgesehen sein. Alternativ kann die Servereinrichtung auch über eine Netzwerkverbindung mit dem System verbunden sein, was eine räumliche Entkopplung zwischen der zentralen Recheneinheit und den sonstigen Systemkomponenten ermöglicht. Darüber hinaus kann die zentrale Recheneinheit auch als Cloud-Computing-Dienst, ohne eine lokale oder dezentrale dem System permanent zuordenbare physische Systemkomponente realisierbar sein.

Bezüglich der Erfassung von Zuständen der Raumeinheiten kommen bevorzugt Sensoreinheiten zum Einsatz, die zur Erfassung von Mikrobewegungen eingerichtet sind. Alternativ oder zusätzlich können jedoch auch andere Sensoreinheiten zum Einsatz kommen. Dazu zählen unter anderem Sensoreinheiten, die einen Abbildungssensor oder einen Kamerasensor umfassen, der zumindest einen Teil einer Raumeinheit abbildet. Dabei können unterschiedliche Spektralbereiche des elektromagnetischen Spektrums von Interesse sein und folglich die Abbildungs- oder Kamerasensoren für eine Erfassung im jeweiligen Wellenlängenbereich eingerichtet sein. Beispielsweise können Infrarot-Bildgebungssensoren zum Einsatz kommen. Zusätzlich oder alternativ können Sensoreinheiten zum Einsatz kommen, die eine 2,5-dimensionale oder 3-dimensionale Erfassung der Raumeinheit ermöglichen. Durch derartige Scanner wird ein Höhenraster oder eine Punktewolke generiert, aus denen eine Veränderung der Raumeinheit oder von Zuständen der Raumeinheit gut und zuverlässig erfasst werden kann/können. Die Sensoreinheiten können in dieser Ausgestaltung beispielsweise als Stereo-Laser-Scanner ausgebildet sein.

Besonders bevorzugt kann eine Sensoreinheit sowohl zur Erfassung von räumlichen und/oder optischen Eigenschaften der Raumeinheit, als auch zur Erfassung von Mikrobewegungen in der Raumeinheit eingerichtet sein. Dadurch wird beispielsweise eine Erfassung ermöglicht, die erlaubt festzustellen, wo, in welcher Lage mit welcher Bewegung/Beschleunigung ein Lebewesen oder ein Mensch in der Raumeinheit anwesend ist oder sich in der Raumeinheit bewegt. Dazu können die vorangehend beschriebenen Ausgestaltungen der Sensoreinheit in bevorzugter Weise miteinander verknüpft und kombiniert werden.

Nachfolgend wird das erfindungsgemäße System und auch das erfindungsgemäße Verfahren im Wesentlichen anhand von Sensoreinheiten beschrieben, die eine Erfassung von Mikrobewegungen ermöglichen oder die Erfassung von Mikrobewegungen in den Vordergrund stellen. Aus bereits beschriebenen Ausgestaltungen der Sensoreinheiten mit anderen oder ggf. zusätzlichen Erfassungseigenschaften, als bei Sensoreinheiten, die als Abbildungseinheit oder als Raumscanner fungieren, lassen sich jedoch ebenfalls die nachfolgend beschriebenen vorteilhaften Ausgestaltungen und deren Vorteile erzielen oder gar noch verbessern.

Erfindungsgemäß ist vorgesehen, dass die Sensormodule mit einer Mikrobewegungs-Sensoreinheit neben der Sensoreinheit zumindest eine weitere Funktionseinheit aufweisen. Dadurch wird in besonders vorteilhafter Weise ermöglicht, dass das Sensorsystem, insbesondere die den Raumeinheiten zugeordneten Sensormodule in bestehende Infrastruktur, insbesondere in bestehende Gebäudeinfrastruktur oder Infrastruktur für Gebäudemanagement integriert werden können. Bei den Funktionseinheiten handelt - es sich um andere Sensoren, nämlich einen Bewegungssensor und einen Rauch-Detektor, insbesondere Sensoren zur Erfassung anderer Eigenschaften oder Zustände. Beispielsweise kann die zumindest eine weitere Funktionseinheit als Helligkeitssensor, Löschwasser-Sprinkler oder dgl. ausgeführt sein. Ein weiterer Vorteil der Kombination der Sensormodule umfassend eine Mikrobewegungs-Sensoreinheit und eine weitere Funktionseinheit, liegt darin, dass ein besonders vorteilhaftes Nachrüsten der Sensormodule ermöglicht wird, indem beispielsweise eine ursprünglich der Raumeinheit zugeordnete Funktionseinheit durch eine neue Funktionseinheit ersetzt wird, die jedoch neben der Funktionseinheit selbst die Sensoreinheit, also die Mikrobewegungs-Sensoreinheit umfasst und somit nicht mehr nur noch als Funktionseinheit sondern als Sensormodul dient. In diesem Zusammenhang kann es auch besonders vorteilhaft sein, wenn die Verbindung zwischen der Sensoreinheit und der zentralen Recheneinheit drahtlos ausgeführt ist. Denn dadurch wird eine Nachrüstung ermöglicht, die, zumindest auf physischer bzw. gegenständlicher Ebene keine Nachrüstung von Verbindungsinfrastruktur in dem Bereich erfordert, in dem das Sensormodul angeordnet werden soll und gegebenenfalls eine ehemalige Funktionseinheit ersetzen bzw. ergänzen soll. Beispielsweise kann bei einer Integration der Mikrobewegungs-Sensoreinheit in einen ansonsten nicht vernetzten, insbesondere akkubetriebenen, Rauchmelder und einer entsprechenden drahtlosen Verbindung zwischen der Sensoreinheit und der zentralen Recheneinheit ein einfaches, schnelles und kostengünstiges Ersetzen oder Nachrüsten des Sensormoduls erfolgen und damit die besonderen Funktionalitäten des erfindungsgemäßen Gebäude-Sensorsystems bereitstellen, ohne dass ein merklicher Mehraufwand im Rahmen der Installation des Sensormoduls betrieben werden muss.

Im Zusammenhang mit der Nachrüstbarkeit der Sensormodule kann zudem besonders vorteilhaft vorgesehen sein, wenn gegebenenfalls bereits vorhandene Infrastruktur der Raumeinheiten weiterbenutzt werden kann. In diesem Zusammenhang bietet sich besonders an, dass möglichst die für eine spezielle Funktionseinheit bereits vorhandene Infrastruktur von einem entsprechenden Sensormodul mitbenutzt oder aufgegriffen wird. Dementsprechend sieht eine weitere, besonders vorteilhafte Ausführungsform des Sensorsystems vor, dass die Sensormodule mit einer Mikrobewegungs-Sensoreinheit ein Gehäuse umfassen, dessen Form sich nach der zumindest einen weiteren Funktionseinheit richtet. Dementsprechend kann erreicht werden, dass die Infrastruktur der jeweiligen Funktionseinheit, welche durch die Sensoreinheit ergänzt wird, weiter genutzt oder mitbenutzt wird. In dem relativ trivialen, oben bereits genannten Beispiel eines Rauchmelders, welcher durch die Mikrobewegungs-Sensoreinheit ergänzt wird, kann dies beispielsweise dazu führen, dass mit dem Aufgreifen oder Nachbilden des entsprechenden Gehäuses der Funktionseinheit eine Montagevorrichtung, beispielsweise eine in der Raumeinheit befestigte Montageplatte des Rauchmelders, für das Sensormodul weiterverwendet werden kann, wodurch die nachträgliche Einrichtung oder die nachträgliche Integration des erfindungsgemäßen Gebäude-Sensorsystems besonders erleichtert wird.

Gemäß einer weiteren, besonders vorteilhaften Ausgestaltung des Sensorsystems kann vorgesehen sein, dass die Sensoreinheit einen Radarsensor, insbesondere einen Ultra-Wide-Band (UWB) Sensor umfasst. Derartige Sensoren eignen sich aus verschiedenen Gründen besonders als Sensoreinheit zur Erfassung von Mikrobewegungen. Einerseits ermöglichen derartige Sensoreinheiten eine hohe räumliche Auflösung. Andererseits wird von derartigen Sensoreinheiten sehr wenig Leistung emittiert, sodass sie bedenkenlos im Gebäudeinneren eingesetzt werden kann. Die Sensoreinheit ist besonders bevorzugt als kombinierte Sende- und Empfangseinheit (Transceiver) ausgebildet und ermöglicht das Abstrahlen oder Senden von Strahlung im Radiofrequenzbereich (RF) sowie den Empfang von zeitlich versetzten Reflexsignalen, die von Gegenständen in der Raumeinheit oder Gegenständen der Raumeinheit reflektiert und zurückgesendet werden.

Gemäß einer weiteren besonders bevorzugten Ausgestaltung des Systems ist vorgesehen, dass die Recheneinheit dazu eingerichtet ist, eine Mikrobewegung einer Vitalfunktion eines Lebewesens in der Raumeinheit in den Sensorsignalen zu identifizieren. Dazu kann vorgesehen sein, dass aus den Sendesignalen oder den Empfangssignalen der Sensoreinheit eine Frequenzmodulation bestimmt wird, die als Mikrodopplereffekt bezeichnet wird. Dazu kann beispielsweise eine Dopplerkarte (Doppler-Map) oder eine Doppler-Matrix erstellt werden, die dann gegebenenfalls mittels einer FourierTransformation in einen Frequenzraum übertragen wird und wobei mittels der Erkennung von Grundschwingungen und harmonischen Oberschwingungen oder Obertönen im Frequenzraum eine Vitalfunktion eines Lebewesens in der Raumeinheit identifiziert werden kann.

Ebenfalls kann gemäß einer besonders bevorzugten Ausführungsform des Sensorsystems vorgesehen sein, dass die zentrale Recheneinheit dazu eingerichtet ist eine Atemfrequenz und/oder eine Frequenz eines Herzschlages in den Sensorsignalen zu identifizieren. Alternativ kann dies bereits durch die Sensoreinheit ermöglicht werden. Dazu kann vorgesehen sein, dass die Sensoreinheit eine Sensorrecheneinheit umfasst.

Sowohl die Identifizierung einer Vitalfunktion eines Lebewesens, als auch die besondere Ausgestaltung über die Identifizierung einer Atemfrequenz und/oder einer Frequenz eines Herzschlages ermöglicht die genaue und sichere Erfassung einer Anwesenheit eines Lebewesens, insbesondere eines Menschen in der jeweiligen Raumeinheit. Dies ist für das vorgeschlagene Gebäude-Sensorsystem von besonders hohem Wert, da vielfach das Gebäudemanagement auf eine Anwesenheit oder eine Abwesenheit eines Lebewesens, insbesondere eines Menschen in der Raumeinheit ausgerichtet ist. Mit anderen Worten ausgedrückt bedeutet dies, dass eine Gebäudeautomatisierung und ein integratives Gebäudemanagement ganz besonders davon abhängig sind mit einer hohen Sicherheit und Zuverlässigkeit bestimmen zu können, ob in der jeweiligen Raumeinheit Lebewesen, insbesondere Menschen anwesend sind. Dazu ist besonders die Atmung oder die Atemfrequenz sowie der Pulsschlag und dessen Frequenz ein sicheres und zuverlässiges Entscheidungskriterium oder Identifikationsmittel, sodass sich gerade diese Vitalfunktion besonders eignet um sicher die Anwesenheit oder Abwesenheit sowie das Betreten oder Verlassen eines Menschen in oder aus der Raumeinheit festzustellen. Diese Vitalfunktionen sind auch deshalb besonders vorteilhaft mittels des Sensormoduls, insbesondere mittels der Sensoreinheit zu bestimmen, da in einem noch weitergehenden Integrationsschritt des Gebäudemanagements eine entsprechende Anwendungseinheit neben der schieren Anwesenheit oder Abwesenheit eines Lebewesens bzw. eines Menschen gegebenenfalls sogar in Abhängigkeit des körperlichen Zustands eines anwesenden Menschen, der wiederum über die entsprechenden Vitalfunktionen über Mikrobewegungen bestimmt wird, ein anwendungsspezifisches Ausgabesignal generiert, welches dann in dem Gebäudemanagement bzw. im Betrieb des Gebäudes berücksichtigt oder weiterverarbeitet wird.

Wie oben bereits erwähnt, kann anstatt oder zusätzlich zur Erfassung und Identifizierung von Mikrobewegungen, insbesondere Vitalfunktionen, besonders bevorzugt Herzschlag und/oder Atmung, eine optische Abbildung oder Erfassung und/oder eine räumliche Abbildung oder Erfassung der Raumeinheit mittels entsprechender Sensoreinheiten und eine dazugehörige Identifizierung von Zuständen der Raumeinheit stattfinden. Diese kann/können dann bevorzugt ein Bewegungsprofil der Raumeinheit, insbesondere ein Bewegungsprofil eines Menschen oder Lebewesens als Zustand der Raumeinheit betreffen. Die Identifizierung des Menschen oder Lebewesens kann dabei entweder über eine Mikrobewegungs-Sensoreinheit oder über die optische und/oder räumliche Erfassung mittels der zumindest einen Sensoreinheit erfolgen. Dadurch wird die Auswertung oder die Auswertbarkeit der Informationen durch die Auswertungseinheit weiter verbessert, da in vielerlei Hinsicht die Lokalisierungsinformation zusätzlich zur reinen Erfassung oder Identifizierung eines Lebewesens oder eines Menschen einen wertvollen Informationsmehrwert darstellt, auf dessen Grundlage die anwendungsbezogene Verarbeitung mittels der Anwendungseinheit durchgeführt werden kann.

Nachfolgend wird noch näher auf die Vorrichtungen eingegangen werden, welche mit der Anwendungseinheit verbunden sind oder verbindbar sind und welche das anwendungsspezifische Ausgabesignal weiter verarbeiten.

Gemäß einer ersten vorteilhaften Ausführungsform kann nämlich vorgesehen sein, dass das Sensorsystem eine Steuereinheit zur Steuerung zumindest einer Gebäudefunktion, insbesondere einer Raumeinheitfunktion umfasst, wobei die zumindest eine Anwendungseinheit mit der Steuereinheit verbunden ist und die Steuereinheit dazu eingerichtet ist die Gebäudefunktion insbesondere die Raumeinheitsfunktion, in Abhängigkeit von dem Ausgabesignal der Anwendungseinheit zu steuern.

Als wohl einfachstes Beispiel in diesem Zusammenhang kann als Steuereinheit eine Steuereinheit für eine Klimatisierung einzelner Raumeinheiten oder Gruppen von Raumeinheiten für eine Beleuchtung einzelner Raumeinheiten oder Gruppen von Raumeinheiten und/oder für die Beschallung einzelner Raumeinheiten oder Gruppen von Raumeinheiten herangezogen werden. In diesem Fall kann mittels des vorgeschlagenen Sensorsystems über die mittels der Sensoreinheit und gegebenenfalls der Recheneinheit erfassten und identifizierten Mikrobewegungen auf Veränderungen in den Raumeinheiten sowie auf den jeweiligen Ist-Zustand der Raumeinheiten, insbesondere bezüglich der Anwesenheit von Lebewesen, insbesondere Menschen, geschlossen werden, um dann im Anschluss an eine entsprechende Anfrage und Rückmeldung von bzw. an die Anwendungseinheit eine anwendungsbezogene und raumeinheitsbezogene Auswertung seitens der Anwendungseinheit anzuschließen, die dann mit der Ausgabe eines Ausgabesignals an die Steuereinheit abschließt.

In diesem Fall kann beispielsweise die anwendungsbezogene Verarbeitung der von der Anwendungseinheit erhaltenen Information darin bestehen, dass Informationen hinsichtlich der Anwesenheit von Personen oder Lebewesen in Raumeinheiten mit weiteren anwendungsbezogenen Parametern verglichen oder verknüpft werden. Beispielsweise kann für eine Anwendungseinheit, die mit einer Steuereinheit zur Steuerung der Beleuchtung im Gebäude verbunden ist der weitere anwendungsbezogene Parameter in einer Tageszeit, einer tageszeitbedingten Außenhelligkeit, od.dgl. bestehen. In Abhängigkeit von den weiteren, anwendungsbezogenen Parametern wird folglich von der Anwendungseinheit das Ausgabesignal erzeugt und ausgegeben anhand dessen die Steuereinheit die jeweiligen Gebäudefunktionen, insbesondere Raumeinheitsfunktionen steuern kann. Beispielsweise kann aufgrund des Ausgabesignals seitens der Steuereinheit die Beleuchtung in einzelne Raumeinheiten eingeschaltet oder ausgeschaltet werden. Gleichermaßen kann mittels der Steuereinheit ein Steuersignal zur Klimatisierung der Raumeinheit erzeugt oder ausgegeben werden.

Auch kann dabei vorgesehen sein, dass über die Anwendungseinheit ein übergeordneter Kontext hergestellt wird, der auf der Verallgemeinerung von in einer Raumeinheit identifizierten Mikrobewegungen beruht. Die Verallgemeinerung kann beispielsweise eine räumliche Verallgemeinerung hinsichtlich einer Gruppe von Raumeinheiten betreffen. So kann zum Beispiel eine Steuerung einer Temperatur in einer Gruppe von Raumeinheiten, beispielsweise in den Räumen eines Hotelzimmers, erfolgen sobald entsprechende Mikrobewegungen in einer Raumeinheit identifiziert werden. Gleichermaßen kann auch die Beleuchtung eine räumliche Verallgemeinerung dahingehend stattfinden, dass die Anwendungseinheit ein Ausgabesignal erzeugt, welches durch eine Weiterverarbeitung seitens der Steuereinheit keinen direkten Einfluss auf die Raumeinheit oder die Raumeinheiten ausübt, in denen die Sensoreinheiten angeordnet sind. Zum Beispiel könnte ein Ausgabesignal der Anwendungseinheit die Steuereinheit veranlassen die Beleuchtung auf einem Hotelflur zu dimmen oder auszuschalten, wenn die Sensoreinheiten, welche den Hotelzimmern des Flurs zugeordnet sind keine Anwesenheit eines Lebewesens in den Hotelzimmern detektieren. Dies könnte unabhängig davon erfolgen, ob der Hotelflur als Raumeinheit eine Sensoreinheit aufweist.

Alternativ oder zusätzlich kann vorteilhaft vorgesehen sein, dass das Sensorsystem eine Organisationseinheit umfasst, welche zur Planung von Raumeinheitsarbeiten, insbesondere Wartungs- und Pflegearbeiten eingerichtet ist, wobei eine Anwendungseinheit mit der Organisationseinheit verbunden ist, und die Organisationseinheit dazu eingerichtet ist die Planung von Gebäudearbeiten in Abhängigkeit von dem Ausgabesignal der Anwendungseinheit durchzuführen.

Die Vorteile der Organisationseinheit lassen sich am anschaulichsten anhand der Abläufe in einem Hotelgebäude oder einem Bürogebäude beschreiben. In derartigen Gebäuden und den entsprechenden Raumeinheiten, fallen eine Vielzahl von Gebäudearbeiten an, die jedoch bevorzugt zu Zeiten durchzuführen sind, in denen die Gebäudenutzer oder Gebäudegäste abwesend sind oder durch die Gebäudearbeiten nicht gestört werden. Zu derartigen Arbeiten gehören u.a. Reinigungsarbeiten, Wartung und Instandhaltungsarbeit. In einem solchen Kontext kann der Anwendungsbezug, unter dem die Anwendungseinheit unter Berücksichtigung der erfassten und identifizierten Mikrobewegungen der Ausgabesignale erstellt, beispielsweise in einem Zeitintervall seit einer zuletzt durchgeführten Gebäudearbeit, insbesondere Raumeinheitsarbeit, oder in der Bewertung einer Anwesenheitszeit einer Person oder eines Lebewesens in der jeweiligen Raumeinheit anhand von einem oder mehreren Grenzwerten oder dergleichen liegen.

Die Organisationseinheit umfasst in einem bevorzugten Ausführungsbeispiel eine Ausgabeeinheit, die ihrerseits bevorzugterweise eine optische Ausgabe ermöglicht, die die Organisation oder Planung der Gebäudearbeiten ausgibt oder anzeigt. Dazu kommen sowohl Bildschirme, Drucker oder dergleichen in Betracht. Auch mobile persönliche digitale Geräte, wie beispielsweise Smartphones oder dergleichen können als Ausgabeeinheit der Organisationseinheiten dienen. Somit kann in besonders vorteilhafter Weise beispielsweise die Reinigung von Hotelzimmern oder Büroräumen in einem entsprechenden Gebäude organisiert werden, wobei das Personal, welches die jeweiligen Arbeiten durchführt von der Organisationseinheit optimierte Vorgaben zur Durchführung der Tätigkeiten oder Arbeiten erhält. Es kann also beispielsweise ermöglicht werden, dass die Anwendungseinheit ein Ausgabesignal erzeugt, welches einen Reinigungsplan impliziert, wobei sowohl die Reihenfolge der zu reinigenden Raumeinheiten als auch die Auswahl der zu reinigenden Raumeinheiten in der raumeinheitsbezogenen und anwendungsbezogenen Auswertung der Information der Sensoreinheiten generiert wird und entsprechend die Organisationseinheit in Abhängigkeit vom Ausgangssignal der Anwendungseinheit einen optischen Plan, optische Anweisungen oder anderweitige Ausgaben oder Hinweise erzeugt, um dem Personal die optimierte Durchführung der Arbeiten vorzugeben oder zu planen.

Alternativ oder zusätzlich kann vorteilhaft vorgesehen sein, dass das Sensorsystem eine Serviceeinheit umfasst, welche zur Identifizierung und/oder Planung von Servicediensten, insbesondere Pflege- und/oder Unterhaltungs- und/oder Fitness- und/oder Wellness-Diensten eingerichtet ist, wobei eine Anwendungseinheit mit der Serviceeinheit verbunden ist, und die Serviceeinheit dazu eingerichtet ist die Identifizierung und/oder Planung von Servicediensten in Abhängigkeit von dem Ausgabesignal der Anwendungseinheit durchzuführen. Zudem kann die Serviceeinheit mit zumindest einer Ausgabeeinheit verbunden sein, so dass ein identifiziertes Serviceangebot oder eine geplante Serviceleistung einem Nutzer, also einem in einer Raumeinheit befindlichen Menschen oder Lebewesen ausgegeben oder angeboten wird. Über eine Eingabeeinheit kann vorgesehen sein, dass ein Nutzer ein Serviceangebot oder eine Serviceleistung bestätigen oder verwerfen kann, also eine Rückmeldung an die Serviceeinheit über eine weitere Verbindung mit der Serviceeinheit übermittelt werden kann.

Die Funktionsweise der Serviceeinheit entspricht weitestgehend der Funktionsweise der Organisationseinheit, wobei jedoch die Interaktionsmöglichkeit mit einem Nutzer und dafür ggf. notwendige Aus-und/oder Eingabeeinheiten zweckmäßig sind, da das Serviceangebot im Gegensatz zu den Wartungs- und Pflegearbeiten gerade in Abstimmung mit dem Nutzer angeboten und erbracht werden sollen.

Eine weitere besonders vorteilhafte Ausführungsform sieht vor, dass das Sensorsystem eine Sicherheitseinheit, zum Erzeugen von Sicherheitshinweisen und/oder zum Koordinieren oder Unterstützen von Rettungsmaßnahmen und/oder zur Koordinierung von Wachschutz- oder Polizeieinsätzen eingerichtet ist, wobei zumindest eine Anwendungseinheit mit der Sicherheitseinheit verbunden ist und die Sicherheitseinheit dazu eingerichtet ist in Abhängigkeit von dem Ausgabesignal der Anwendungseinheit die Sicherheitshinweise zu erzeugen und/oder die Rettungsmaßnahmen und/oder Wachschutz- oder Polizeieinsätze zu koordinieren oder zu unterstützen.

In diesem Zusammenhang kann beispielsweise vorgesehen sein, dass die raumeinheitsbezogenen sowie die anwendungsbezogenen Parameter, auf welche sich die Verarbeitung der Anwendungseinheit stützt, die Lokalisierung von Raumeinheiten bezüglich der vorgesehenen Flucht- und Rettungswege, die Erreichbarkeit von Raumeinheiten bezüglich bestimmter Rettungsmittel, die Anordnung und Ausgestaltung von in den Raumeinheiten befindlichen Warn- und Hinweisgebern und dgl. umfasst. Auch kann vorgesehen sein, dass die anwendungsbezogenen und raumeinheitsbezogenen Informationen und Parameter, welche in die Verarbeitung der Anwendungseinheit einfließen von weiteren Sensoren abseits der vorgesehenen Sensoreinheiten zur Erfassung von Mikrobewegung stammen. Im vorliegenden Beispiel kann also auch vorgesehen sein, dass die anwendungs- und/oder raumeinheitsbezogenen Parameter oder Informationen, welche von der Anwendungseinheit berücksichtigt werden, von Rauchmeldern oder weiteren Sensoren stammen, welche in den Raumeinheiten angeordnet sind. Dementsprechend kann die Sicherheitseinheit auf der Basis des Ausgabesignals der Ausgabeeinheit einerseits Sicherheitswarnungen, Evakuierungswarnungen, die Anzeige von Fluchtwegen und dgl. auslösen. Andererseits kann die Sicherheitseinheit den Einsatzkräften von Rettungsdienst, Wachschutz und/oder Polizei jedoch auch Informationen bereitstellen, die darauf abzielen den jeweiligen Einsatz zu erleichtern und zu optimieren. Beispielsweise kann den Einsatzkräften mitgeteilt werden, in welchen Raumeinheiten und unter welchen Bedingungen noch Lebewesen, insbesondere Personen befindlich sind. Um derartige Informationen zu übermitteln und Hinweise oder Warnungen auszugeben kann ebenfalls vorgesehen sein, dass die Sicherheitseinheit mit Anzeige-und/oder Ausgabemitteln sowie mit Kommunikations- und/oder Übertragungsmitteln versehen ist oder verbunden ist.

Eine weitere, besonders vorteilhafte Ausführung des Sensorsystems sieht zudem vor, dass die Anwendungseinheit ein Mustererkennungsmittel umfasst, welches dazu eingerichtet ist, im Rahmen der anwendungsbezogenen und raumeinheitsbezogenen Verarbeitung der Sensorsignale räumliche und/oder zeitliche Muster in den Zuständen und/oder Zustandsänderungen der zumindest einen Raumeinheit zu erkennen, so dass die Anwendungseinheit ein Ausgabesignal erzeugt, welches von einem Muster eines Zustands und/oder einer Zustandsänderung der zumindest einen Raumeinheit abhängig ist. In diesem Zusammenhang sei erwähnt, dass die Mustererkennung, die von dem Mustererkennungsmittel erbracht wird, über die Erkennung oder Identifizierung von Menschen oder Lebewesen in einer Raumeinheit, die im weiteren Sinne auch Mustererkennungen darstellen, hinausgeht. Die Mustererkennungsmittel sind dazu eingerichtet, gerade für identifizierte Menschen oder Lebewesen die entsprechenden Sensorsignale auf das Vorliegen von Mustern hin zu untersuchen. Dabei können bevorzugt Bewegungsmuster, Aktivitätsmuster oder dergleichen gesucht und womöglich identifiziert werden. Dadurch kann in vorteilhafter Weise die anwendungsbezogene Verarbeitung der Informationen seitens der Anwendungseinheit verbessert werden, indem neben den aktuellen Informationen auch vergangene Muster berücksichtigt werden.

Eine weitere besonders vorteilhafte Ausführung des Sensorsystems sieht zudem vor, dass die Anwendungseinheit ein Vorhersagemittel umfasst, welches dazu eingerichtet ist im Rahmen der anwendungsbezogenen und raumeinheitsbezogenen Verarbeitung der Sensorsignale eine Vorhersage über einen zukünftigen Zustand von zumindest einer Raumeinheit anzufertigen, sodass die Anwendungseinheit ein Ausgabesignal erzeugt, welches zumindest auch von einem zukünftigen Zustand der Raumeinheit abhängt. Die Vorhersage kann dabei einerseits mittelbar oder unmittelbar die Abwesenheit oder Anwesenheit eines Lebewesens, insbesondere einer Person in der Raumeinheit betreffen und auf den von der Sensoreinheit erfassten und von der Recheneinheit ausgewerteten Sensorsignalen beruhen. Andererseits kann die Vorhersage jedoch auch andere anwendungsspezifische oder raumeinheitsbezogene Parameter betreffen und anhand von anderen raumeinheitsbezogenen oder anwendungsspezifischen Informationen getroffen werden, welche beispielsweise über andere Sensoren oder Informationssysteme zur Verfügung gestellt werden. Beispielsweise kann also vorgesehen sein, dass, wenn festgestellt wird, dass morgens ein Lebewesen, insbesondere eine Person eine als Schlafraum ausgebildete Raumeinheit verlässt, die Vorhersage oder Annahme getroffen wird, dass die Person oder das Lebewesen als nächstes eine als Toilette oder Badezimmer ausgestaltete Raumeinheit betritt oder benutzt. In diesem Fall kann die Vorhersage von der jeweiligen Anwendungseinheit beispielsweise genutzt werden, um einen vorläufigen Beleuchtungszustand herzustellen, um beispielsweise eine Belüftung oder Klimatisierung zu aktivieren oder dergleichen. Dementsprechend kann das von der Anwendungseinheit erzeugte Ausgabesignal unter Berücksichtigung der Vorhersage erzeugt werden.

Besonders vorteilhaft kann vorgesehen sein, dass das Vorhersagemittel mit einem Mustererkennungsmittel verknüpft oder verbunden ist und die Vorhersage auf der Basis von mittels der Mustererkennungsmittel identifizierten Mustern durchgeführt wird. Dadurch wird in vorteilhafter Weise ermöglicht, dass die Vorhersage sich nicht nur auf einen aktuellen Zustand stützt, sondern auch vergangene Zustände und darin erkannte Muster mitberücksichtigt.

Die eingangs genannte Aufgabe wird bei dem erfindungsgemäßen Verfahren für ein integratives Gebäudemanagement mittels eines Gebäude-Sensorsystems, welches eine Vielzahl von Sensormodulen umfasst, welche jeweils einer Raumeinheit zugeordnet sind und einen Zustand der Raumeinheit erfasst dadurch gelöst, dass eine Vielzahl von Sensormodulen eine Sensoreinheit, insbesondere zur Erfassung von Mikrobewegungen umfasst, wobei jede Sensoreinheit mit einem Kommunikationsnetzwerk verbunden ist, über welches das Sensormodul mit zumindest einer zentralen Recheneinheit kommuniziert, welche insbesondere Mikrobewegungen in den Sensorsignalen der Sensoreinheiten identifiziert, wobei die zentrale Recheneinheit mit zumindest einer Anwendungseinheit verbunden ist und die Anwendungseinheiten Anfragen, insbesondere über Mikrobewegungen, in den Raumeinheiten an die zentrale Recheneinheit sendet, Informationen, insbesondere über die Mikrobewegungen, der Raumeinheiten empfängt, und die empfangenen Informationen, insbesondere über die Mikrobewegungen anwendungsbezogen und raumeinheitsbezogen verarbeitet und ein anwendungsspezifisches Ausgabesignal erzeugt.

Durch das erfindungsgemäße Verfahren wird einerseits ermöglicht, dass mit hoher Präzision und hoher räumlicher Auflösung Veränderungen in den Raumeinheiten, insbesondere betreffend der Präsenz oder Abwesenheit von Menschen oder Lebewesen erreicht wird und gleichzeitig über eine anwendungsbezogene und raumeinheitsspezifische Weiterverarbeitung oder Aufbereitung der präzisen Informationen ein intelligentes, und ressourcenschonendes Gebäudemanagement für eine Vielzahl von Raumeinheiten erreicht wird. Wie oben bereits beschrieben können dazu alternativ oder zusätzlich auch andere Sensoreinheiten zum Einsatz kommen, die beispielsweise die Raumeinheit optisch oder räumlich erfassen und ggf. die Lage, Bewegung und/oder Beschleunigung von Lebewesen erfassen und womöglich Bewegungsprofile identifizieren.

Bezüglich der Vorteile, der Wirkungen und der Durchführung des Verfahrens wird auch auf die vorangehende Beschreibung des Systems verwiesen, da bei dem Verfahren oder der Durchführung des Verfahrens identische Vorteile realisiert, identische Mittel zum Einsatz kommen und auch weitestgehend die Durchführung des Verfahrens selbst bereits mit Bezug auf die Offenbarung des Systems erfolgt ist oder sich daraus erschließt.

Besonders bevorzugt kann vorgesehen sein, dass die Recheneinheit eine Mikrobewegung einer Vitalfunktion eines Lebewesens in einer Raumeinheit in den Sensorsignalen identifiziert. Dies kann beispielsweise derart erfolgen, dass Signale und reflektierte Antwort-Signale eines UWB-Transreceivers auf das Vorhandensein von Mikro-Dopplersignale untersucht wird, wobei über eine Transformation der ausgewerteten Signale in einem Frequenzraum und eine Untersuchung auf Grundfrequenzen und Oberschwingungen die Vitalfunktionen identifiziert werden können.

Besonders vorteilhaft kann vorgesehen sein, dass die Recheneinheit eine Atemfrequenz und/oder eine Frequenz eines Herzschlags in den Sensorsignalen identifiziert. Dadurch wird besonders vorteilhaft ermöglicht mit hoher Sicherheit die Anwesenheit eines Lebewesens in der jeweiligen Raumeinheit zu identifizieren.

Es kann alternativ vorgesehen sein, dass die Identifizierung von Vitalfunktionen und/oder Herzschlag und/oder Atmung und/oder Lage und/oder Bewegung und/oder Beschleunigung durch eine von der Sensoreinheit oder dem Sensormodul umfassten Recheneinheit durchgeführt wird, wodurch Belastung oder Auslastung der zentralen Recheneinheit verringert wird.

Zudem kann besonders vorteilhaft vorgesehen sein, dass eine Anwendungseinheit mit einer Steuereinheit zur Steuerung zumindest einer Gebäudefunktion, insbesondere einer Raumeinheitsfunktion verbunden ist und die Steuereinheit die Gebäudefunktion insbesondere Raumeinheitsfunktionen, in Abhängigkeit von dem Ausgabesignal der Anwendungseinheit steuert. Dabei kann bevorzugt vorgesehen sein, dass die anwendungsbezogene und raumeinheitsspezifische Auswertung seitens der Anwendungseinheit zusätzlich Informationen und Parameter berücksichtigt, die der Anwendungseinheit entweder statisch oder veränderlich zur Verfügung gestellt sind oder aber der Anwendungseinheit von weiteren Sensoren oder Informationssystemen zur Verfügung gestellt werden. Die Steuerung der Raumeinheitsfunktion oder der Gebäudefunktion kann beispielsweise die Steuerung von Beleuchtung, die Steuerung von Klimatisierung oder die Steuerung von Fahrstühlen sowie die Steuerung von Elektronik oder elektrischen Einrichtungen der Raumeinheit betreffen. So kann die Steuereinheit beispielsweise veranlassen, dass ab einer gewissen Tageszeit, bevorzugt abends oder nachts, nach einer gewissen Zeit, zu der eine Person eine Raumeinheit verlassen hat, die Beleuchtung in der Raumeinheit ausgeschaltet wird, noch im Betrieb befindliche elektrische Geräte oder Elektronik ausgeschaltet oder in einen Ruhezustand versetzt werden und ggf. die Temperatur abgesenkt und die Belüftung reduziert wird. Dazu kann beispielsweise die Anwendungseinheit die Informationen bezüglich der Anwesenheit oder Abwesenheit von Lebewesen, insbesondere Menschen in den jeweiligen Raumeinheiten mit Informationen über die Tagezeit, Informationen über den Ist-Zustand von Gebäudefunktionen oder Vorgaben zum Soll-Zustand von Gebäudefunktionen verknüpfen und ein entsprechendes Ausgabesignal für die Steuereinheit erzeugen.

Ebenfalls kann besonders vorteilhaft vorgesehen sein, dass die Anwendungseinheit mit einer Organisationseinheit verbunden ist, welche zur Planung von Raumeinheitsarbeiten, insbesondere Wartungs- und Pflegearbeiten, eingerichtet ist, und die Organisationseinheit die Planung von Gebäudearbeiten in Abhängigkeit von dem Ausgabesignal der Anwendungseinheit durchführt. Das Ausgangssignal der Anwendungseinheit kann beispielsweise zum Ausdruck bringen, in welchen Raumeinheiten eines Gebäudes, welche als Schlafzimmer ausgestaltet sind, in der vergangenen Nacht, Lebewesen, insbesondere Menschen befindlich waren, die sich zum jetzigen Zeitpunkt bereits nicht mehr in der entsprechenden Raumeinheit aufhalten und bevorzugt auch angrenzende Raumeinheiten bereits verlassen haben, sodass Raumeinheitsarbeiten, wie beispielsweise Reinigungsarbeiten oder Hauskeeping-Arbeit in der entsprechenden als Schlafzimmer ausgestalteten Raumeinheit und an den angrenzenden Raumeinheiten bereits durchgeführt werden können, wo hingegen das Ausgabesignal der Anwendungseinheit für Raumeinheiten, in denen noch Lebewesen, insbesondere Menschen befindlich sind, ein Ausgabesignal erzeugt, welches die Organisationseinheit veranlasst, die entsprechenden Raumeinheitsarbeiten zu unterdrücken oder aufzuschieben, insbesondere Reinigungsarbeiten oder Hauskeeping-Arbeiten aufzuschieben.

Alternativ oder zusätzlich kann vorteilhaft vorgesehen sein, dass das Sensorsystem eine Serviceeinheit umfasst, welche zur Identifizierung und/oder Planung von Servicediensten, insbesondere Pflege- und/oder Unterhaltungs- und/oder Fitness- und/oder Wellness-Diensten eingerichtet ist, wobei eine Anwendungseinheit mit der Serviceeinheit verbunden ist, und die Serviceeinheit Servicedienste in Abhängigkeit von dem Ausgabesignal der Anwendungseinheit identifiziert oder plant. Zudem kann die Serviceeinheit mit zumindest einer Ausgabeeinheit verbunden sein, so dass ein identifiziertes Serviceangebot oder eine geplante Serviceleistung einem Nutzer, also einem in einer Raumeinheit befindlichen Menschen oder Lebewesen ausgegeben oder angeboten wird. Über eine Eingabeeinheit kann vorgesehen sein, dass ein Nutzer ein Serviceangebot oder eine Serviceleistung bestätigen oder verwerfen kann, also eine Rückmeldung an die Serviceeinheit über eine weitere Verbindung mit der Serviceeinheit übermittelt werden kann.

Die Funktionsweise der Serviceeinheit entspricht weitestgehend der Funktionsweise der Organisationseinheit, wobei jedoch die Interaktionsmöglichkeit mit einem Nutzer und dafür ggf. notwendige Aus-und/oder Eingabeeinheiten zweckmäßig sind, da das Serviceangebot im Gegensatz zu den Wartungs- und Pflegearbeiten gerade in Abstimmung mit dem Nutzer angeboten und erbracht werden soll.

Ebenfalls kann vorteilhaft vorgesehen sein, dass eine Anwendungseinheit mit einer Sicherheitseinheit verbunden ist, welche zum Erzeugen von Sicherheitshinweisen und/oder zum Koordinieren oder Unterstützen von Rettungsmaßnahmen und/oder zur Koordinierung von Wachschutz- oder Polizeieinsätzen eingerichtet ist, und die Sicherheitseinheit in Abhängigkeit von dem Ausgabesignal der Anwendungseinheit die Sicherheitshinweise erzeugt und/oder die Rettungsmaßnahmen und/oder Wachschutz- oder Polizeieinsätze koordiniert oder unterstützt. Die Anwendungseinheit kann also beispielsweise ein Ausgabesignal erzeugen, welche angibt, ob in einer entsprechenden Raumeinheit ein Lebewesen befindlich ist, ob in der jeweiligen Raumeinheit eine erhöhte Raumtemperatur und ggf. eine Rauchentwicklung vorliegt, ob auf einem Fluchtweg der jeweiligen Raumeinheit Bereiche mit einer erhöhten Temperatur oder einer Rauchentwicklung befindlich sind, ob die jeweilige Raumeinheit mit speziellen Rettungsmitteln, wie beispielsweise einer Rettungsleiter erreichbar ist. Darüber hinaus kann ggf. auch neben der schieren Anwesenheit oder Abwesenheit eines Lebewesens in der Raumeinheit die mittels der Sensoreinheiten erfassten und von der Recheneinheit identifizierten Signale, insbesondere die identifizierten Vitalfunktionen hinsichtlich des Vitalzustands einer in der Raumeinheit befindlichen Person ausgewertet werden. Erfindungsgemäß wird in Abhängigkeit dessen sowie in Abhängigkeit der weiteren vorangehend genannten Parametern im Ausgabesignal der Anwendungseinheit eine Evakuierung- oder Rettungsdringlichkeitsstufe für das jeweilige Lebewesen bzw. für die jeweilige Raumeinheit festgelegt.

Entsprechend der jeweiligen Ausgabesignale kann dann mittels der Sicherheitseinheit eine Ausgabe von Sicherheitshinweisen erzeugt oder veranlasst werden sowie die Rettungsmaßnahmen, die Wachschutz- oder Polizeieinsätze sowie vergleichbare Einsätze von Einsatzkräften über die Bereitstellung entsprechender Informationen, beispielsweise in digitaler Form oder in Form von Anzeigen oder Ausgaben, unterstützt oder koordiniert werden. So kann beispielsweise vorgesehen sein, dass die Sicherheitseinheit eine Verbindung zu Kommunikationsmitteln der Einsatzkräfte aufweist und die Informationen zur Unterstützung oder Koordinierung der Maßnahmen oder Einsätze unmittelbar an die Einsätzkräfte übermittelt. Alternativ kann auch vorgesehen sein, dass die entsprechenden Informationen zentral oder dezentral im Gebäude ausgegeben, insbesondere angezeigt oder ausgedruckt werden. Alternativ kann auch vorgesehen sein, dass die Sicherheitseinheit mit zentralen Leitstellen der Einsatzkräfte verbunden ist und die Informationen zur Koordinierung oder Unterstützung der Einsätze und Maßnahmen an die Leitstellen übermittelt werden.

Ebenfalls kann für das Verfahren vorgesehen sein, dass die Anwendungseinheit ein Mustererkennungsmittel umfasst, welches im Rahmen der anwendungsbezogenen und raumeinheitsbezogenen Verarbeitung der Sensorsignale räumliche und/oder zeitliche Muster in den Zuständen und/oder Zustandsänderungen der zumindest einen Raumeinheit erkennt, wobei die Anwendungseinheit ein Ausgabesignal erzeugt, welches von einem Muster eines Zustands und/oder einer Zustandsänderung der zumindest einen Raumeinheit abhängt. Beispielsweise können die Mustererkennungsmittel ein Anwesenheits-und/oder Abwesenheitsmuster über einen gewissen Zeitraum erkennen. Die Mustererkennungsmittel können jedoch, je nach Ausgestaltung der Sensoreinheiten auch Bewegungsmuster identifizieren. Auch kann besonders vorteilhaft vorgesehen sein, dass die Muster eine Vielzahl von Raumeinheiten und deren Zustände betreffen. Beispielsweise kann ein Muster auch eine überwiegende Abwesenheit von Lebewesen in den Raumeinheiten für einen gewissen täglichen oder anderweitig periodischen Zeitraum betreffen, so dass die Mustererkennungsmittel in der Lage sind festzustellen, ob beispielsweise einmalig oder wiederkehrend 80% oder 90% mit den Sensoreinheiten erfassten Raumeinheiten keine Anwesenheit eines Lebewesens vorliegt.

Eine weitere besonders vorteilhafte Ausgestaltung des Verfahrens sieht vor, dass die Anwendungseinheit ein Vorhersagemittel umfasst, welches im Rahmen der anwendungsbezogenen und raumeinheitsbezogenen Verarbeitung der Sensorsignale eine Vorhersage über einen zukünftigen Zustand von zumindest einer Raumeinheit anfertigt, wobei die Anwendungseinheit ein Ausgabesignal erzeugt, welches von einem zukünftigen Zustand der Raumeinheit abhängt. Dabei kann es sich einerseits um eine Vorhersage der Anwesenheit oder Präsenz eines Lebewesens, insbesondere eines Menschen in der jeweiligen Raumeinheit handeln und andererseits kann auch eine Vorhersage bzgl. eines sonstigen, mit der Raumeinheit verknüpften oder verknüpfbaren Parameters stattfinden. Beispielsweise kann anhand von aktuellen und vergangenen Temperaturwerten auf die zukünftige Temperatur in einer Raumeinheit geschlossen oder die zukünftige Temperatur einer Raumeinheit vorhergesagt und bei der Erzeugung des Ausgabesignals der Anwendungseinheit berücksichtigt werden, um beispielsweise die Steuereinheit zur Steuerung einer Klimatisierung mit einem optimierten Ausgabesignal zu versorgen, sodass die Steuereinheit die Gebäudefunktion, insbesondere die Klimatisierung der Raumeinheit optimiert steuern oder betreiben kann.

Besonders bevorzugt berücksichtigt ist die Vorhersage die mit den Mustererkennungsmitteln erkannten Muster.

In der bisherigen Beschreibung des Systems sowie des Verfahrens wurde durchgängig auf eine Vielzahl von Sensormodulen sowie eine Vielzahl von Sensoreinheiten abgestellt. In einem Gebäudekontext, in dem besonders viele Raumeinheiten überwacht und/oder analysiert werden sollen, bietet ein derartiges System sowie ein derartiges Verfahren einen besonders großen Mehrwert. Dies ist auch dem Umstand geschuldet, dass mit einer steigenden Anzahl an Sensoreinheiten, und damit einer wachsenden Menge an zur Verfügung stehenden Informationen, mittels der Anwendungseinheit und den Vorrichtungskomponenten, die das Ausgangssignal der Anwendungseinheit erhalten, besonders zuverlässige und weitreichende Feststellungen getroffen *werden können* und das Gebäudemanagement anhand der Feststellungen ausgerichtet werden kann. Nichtsdestotrotz ist ein erfindungsgemäßer Mehrwert auch bereits erreichbar, wenn das System und/oder das Verfahren mit lediglich einem Sensormodul und/oder einer Sensoreinheit ausgestattet ist oder betrieben wird. Denn bereits bei der anwendungsbezogenen und raumeinheitsbezogenen Verarbeitung der Sensorsignale mittels der Anwendungseinheit kann für eine einzelne Sensoreinheit und dementsprechend eine einzelne Raumeinheit ein vorteilhaftes, integratives Management der Raumeinheit oder des Gebäudes erreicht werden.

Die Erfindung betrifft weiter eine Beherbergungseinrichtung, insbesondere ein Hotel, umfassend ein oben beschriebenes Sensorsystem, wobei die Raumeinheiten zumindest ein Hotelzimmer und/oder einen Raum eines Hotelzimmers umfassen. Die besondere Stärke des erfindungsgemäßen Systems und damit auch die besondere Weiterentwicklung bezüglich der Beherbergungseinrichtung liegt insbesondere darin, dass für die Raumeinheiten, denen eine Sensoreinheit zugeordnet ist, mit ausgesprochen hoher Zuverlässigkeit die Anwesenheit oder die Abwesenheit eines Lebewesens, insbesondere eines Menschen erkannt und festgestellt werden kann, unabhängig von sonstigen Faktoren, wie beispielsweise Bewegung, Lichtverhältnissen od.dgl. Dies wird insbesondere dann erreicht, wenn die Sensoreinheiten zur Erfassung von Mikrobewegungen, insbesondere zur Erfassung von Vitalfunktionen eingerichtet und ausgebildet sind. Durch die Abwesenheitserkennung oder Anwesenheitserkennung kann jedoch das Gebäudemanagement und damit die Behebungseinrichtung erheblich verbessert werden. Beispielsweise kann in einem Schlafraum eines Hotelzimmers, welcher mit einer Sensoreinheit des erfindungsgemäßen Sensorsystems überwacht wird, eine automatische Licht- oder Beleuchtungssteuerung durch das System realisiert werden, deren Auslöser oder Steuergröße die tatsächliche Anwesenheit oder Abwesenheit eines Lebewesens, insbesondere eines Menschen ausmacht, sodass im Gegensatz zu bekannten Beherbergungseinrichtungen und deren Systemen zur Gebäudesteuerung oder zum Gebäudemanagement auch bei ruhenden oder schlafenden Gästen ein zuverlässiges und korrektes Gebäudemanagement erfolgen kann. Neben einem Hotel können als Beherbergungseinrichtungen auch Schiffe, beispielsweise Kreuzfahrtschiffe oder andere Personenschiffe in Betracht kommen. Auch behördliche oder staatliche Beherbergungseinrichtungen, wie beispielsweise Justizvollzugsanstalten od.dgl. können erfindungsgemäße Beherbergungseinrichtungen darstellen. In diesem Zusammenhang kann beispielsweise eine Zelle als Raumeinheit von einem Sensormodul und/oder eine Sensoreinheit erfasst und/oder überwacht werden.

Eine ebenfalls von der Erfindung umfasste Stallungseinrichtung, insbesondere ein Haus- und/oder Nutztierstall, besonders bevorzugt ein Pferdestall, umfassend ein oben beschriebenes Sensorsystem kann dadurch realisiert werden, dass die Raumeinheiten zumindest einen Tieraufenthaltsbereich, insbesondere eine Pferdebox umfassen. Für die erfindungsgemäße Stallungseinrichtung kann durch die Integration des beschriebenen Sensorsystems in besonders vorteilhafter Weise erreicht werden, dass das Gebäudemanagement in einer bisher ungekannten Weise erweitert und verbessert wird. Neben einer automatisierten Ausgabe von Futtermitteln, die beispielsweise über eine mit der Anwendungseinheit verbundene Steuereinheit realisiert werden kann, sowie einer automatisierten oder optimiert geplanten Reinigung der Tiefaufenthaltsbereiche, insbesondere Pferdebox, kann auch die Erfassung und Überwachung von Vitalfunktionen besonders vorteilhaft für das Gebäudemanagement genutzt werden. Dabei kann neben einer besonders zuverlässigen Erkennung einer Anwesenheit oder einer Abwesenheit eines Tiers, insbesondere eines Pferds in dem Aufenthaltsbereich gerade bei einer Anwesenheit der gesundheitliche Zustand des Tiers erfasst werden und in das Gebäudemanagement mit einbezogen werden. So ist es beispielsweise möglich, dass aufgrund der Vitalfunktion Koliken oder ähnliche Erkrankungen bei Pferden oder vergleichbaren Tieren erkannt werden und eine ggf. vorgesehene Sicherheitseinheit des Systems Sicherheitshinweise erzeugt, beispielsweise um in der Stallungseinrichtung anwesendes Personal zu alarmieren, oder um ggf. externe Stellen oder Personen auf die veränderten Vitalfunktionen oder die erkannte Erkrankung, beispielsweise veterinärärztliches Personal, aufmerksam zu machen oder zu alarmieren. Gleiches gilt beispielsweise für eine in der Stallungseinrichtung stattfindende Geburt. Hier kann durch die Erfassung von Vitalfunktionen, also beispielsweise durch die Erfassung einer neuen oder zusätzlichen, separaten Vitalfunktion in einer Raumeinheit, in der bisher schon eine Vitalfunktion erfasst wurde, durch die Anwendungseinheit und die mit der Anwendungseinheit verbundenen und mit dem Ausgangssignal der Anwendungseinheit beaufschlagten Systemkomponenten auf eine Geburt geschlossen und entsprechende Gebäudemanagementvorkehrungen und Abläufe ausgelöst und/oder durchgeführt werden.

Für eine erfindungsgemäße medizinische Unterbringungseinrichtung, insbesondere ein Krankenhaus und/oder Alters- und/oder Pflegeheim mit einem oben beschriebenen, erfindungsgemäßen Sensorsystem, bei dem die Raumeinheiten zumindest einen Patienten- oder Bewohnerlagerungsbereich, insbesondere ein Patienten- oder Bewohnerbett umfassen, kann in besonders vorteilhafter Weise ein mehrstufiges Gebäudemanagement erreicht werden. Wie oben bereits in einem verwandten Zusammenhang beschrieben, wird durch die Sensoreinheiten des erfindungsgemäßen Systems, insbesondere wenn diese zur Erfassung von Mikrobewegungen und zur Erkennung von Vitalfunktionen eingerichtet und ausgebildet sind, ermöglicht, dass mit besonders hoher Zuverlässigkeit die tatsächliche Abwesenheit oder Anwesenheit eines Menschen, insbesondere eines Patienten oder eines Bewohners in der Raumeinheit erfasst wird. Dadurch kann in einer ersten Stufe des Gebäudemanagements allein schon aufgrund der jeweils festgestellten Anwesenheit oder Abwesenheit sowie aufgrund der Veränderungen der überwachten Raumeinheiten bezüglich Anwesenheit und Abwesenheit ein besonders vorteilhaftes und weitreichendes Gebäudemanagement realisiert werden. Beispielsweise kann, vorteilhafter Weise unter Einbeziehung eines Mustererkennungsmittels und/oder eines Vorhersagemittels der Anwendungseinheit des Systems ein Anwesenheitsverlauf oder eine Anwesenheitsänderung über verschiedene Raumeinheiten hinweg erkannt, ausgewertet und basierend darauf geeignete Maßnahmen eingeleitet werden. Für ein Alters- und/oder Pflegeheim kann beispielsweise die ununterbrochene Anwesenheit eines Patienten oder Bewohners in einem Lagerungsbereich erfasst und ausgewertet werden, um durch das System ggf. Maßnahmen zu ergreifen oder zu veranlassen, die eine gewisse Mindestmobilisierung der Bewohner und Patienten bewirken. Dazu kann neben der Sicherheitseinheit des Systems auch die Organisationseinheit oder die Steuereinheit des Systems zum Einsatz kommen. Beispielsweise kann vorgesehen sein, dass ein Patientenbett oder Bewohnerbett mittels der Steuereinheit automatisch gesteuert wird, um einem Bewohner oder Patienten das Aufstehen oder Verlassen des Bettes zu erleichtern. Alternativ oder zusätzlich kann über die Organisationseinheit oder die Sicherheitseinheit das Pflegepersonal auf eine notwendige Mobilisierung eines Bewohners oder Patienten aufmerksam gemacht sowie die entsprechenden Tätigkeiten vorteilhaft oder optimiert organisiert werden.

In ähnlicher Weise könnte in der erfindungsgemäßen medizinischen Unterbringungseinrichtung die ununterbrochene Anwesenheitszeit in einer als Sanitärraum oder Sanitäreinheit ausgebildeten Raumeinheit erfasst und/oder ausgewertet werden und durch das System auch in diesem Zusammenhang Hinweise erzeugt und/oder Maßnahmen ergriffen werden können, um einem Patienten oder Bewohner beim Überschreiten einer vordefinierten maximalen Aufenthaltszeit oder Anwesenheitszeit in einem Sanitärraum oder Sanitärbereich die notwendige Unterstützung und/oder Hilfe zukommen zu lassen.

In einer weiteren Stufe des Gebäudemanagements könnte im Rahmen einer medizinischen Unterbringungseinrichtung das System neben der reinen Anwesenheit oder Abwesenheit eines Lebewesens, insbesondere Bewohnern oder Patienten, in einer Raumeinheit auch die erfassten Mikrobewegungen, insbesondere die erfassten und identifizierten Vitalfunktionen auswerten, sodass beispielsweise die von einer Sicherheitseinheit erzeugten Sicherheitshinweise aus den Vitalfunktionen abgeleitete medizinische Informationen umfassen oder aufweisen, die vom medizinischen Personal bei der Diagnose und/oder Therapie der Patienten oder Bewohner genutzt werden können.

Bei einer erfindungsgemäßen Tagungs- und/oder Konferenzeinrichtung, welche das erfindungsgemäße System umfasst, wobei die Raumeinheiten zumindest einen Tagungs- und/oder Konferenzraum umfass*t*, können im Wesentlichen die mit Bezug auf die Beherbergungseinrichtung beschriebenen Vorteile erreicht werden. Durch das sichere Wissen über die aktuelle Nutzung der Tagungs- und/oder Konferenzräume, insbesondere durch das sichere Wissen über die Anwesenheit oder die Abwesenheit von Lebewesen oder Menschen, insbesondere auch durch die zuverlässige Erfassung der Personenzahl, kann eine verbesserte Steuerung der Raumfunktionen ermöglicht werden. Gleichermaßen kann durch das System, insbesondere bei einem langfristigen Einsatz, beispielsweise mittels der Organisationseinheit eine auf der Grundlage einer statistischen Auswertung, beispielsweise mittels der Anwendungseinheit, eine optimierte Organisation der Tagungs- und/oder Konferenzeinrichtung erreicht werden.

Bei einer erfindungsgemäßen Arbeitsstätte, insbesondere Büroeinheit, mit einer Vielzahl von Arbeitsplätzen, welche ein oben beschriebenes erfindungsgemäßes Sensorsystem umfasst und bei der die Raumeinheiten zumindest einen einem Arbeitsplatz zugeordneten Raum oder Bereich der Arbeitsstätte umfassen, lassen sich vom Grundgedanken ähnliche Vorteile und Weiterentwicklungen realisieren, wie bei den erfindungsgemäßen Beherbergungseinrichtungen und Tagungs- und/oder Konferenzeinrichtungen. Auch hier ist insbesondere die sehr zuverlässige, beispielsweise auf Mikrobewegungen und/oder Vitalfunktionen basierende Erfassung und Erkennung von Anwesenheit oder Abwesenheit eine besonders vorteilhafte Grundlage für das Gebäudemanagement der Arbeitsstätte. Dabei kann über eine Anwendungseinheit des Systems sowie der Ausgangssignale nicht nur die aktuelle Nutzung der Raumeinheiten, also der Arbeitsplätze erfasst und das Gebäudemanagement aufbauend auf der erfassten Nutzung optimiert werden, es kann durch die Anwendungseinheit auch eine statistische Auswertung, insbesondere unter Einbeziehung der Mustererkennungsmittel und der Vorhersagemittel der Anwendungseinheit erreicht werden, die über einen längeren Zeitraum die Nutzung erfasst, die voraussichtliche zukünftige Nutzung bestimmt oder vorhergesagt wird und das Gebäudemanagement daraufhin abgestimmt oder diesbezüglich optimiert wird. Durch die statistische Auswertung seitens der Anwendungseinheit können erhebliche Kosteneinsparungen realisiert werden.

Hinsichtlich der erfindungsgemäßen Verwendungen des erfindungsgemäßen Verfahrens für das Gebäudemanagement einer Behebungseinrichtung, einer Stallungseinrichtung, einer medizinischen Unterbringungseinrichtung, einer Tagungs- und/oder Konferenzeinrichtung und/oder einer Arbeitsstätte wird auf die vorangegangene Beschreibung der entsprechenden Vorrichtungen verwiesen. Die Verwendungen des Verfahrens ermöglichen und realisieren die mit Bezug auf die Vorrichtungen bereits beschriebenen Vorteile und Weiterbildungen des Stands der Technik.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1: eine schematische Darstellung eines Aufbaus eines erfindungsgemäßen Sensorsystems;
- Fig. 2: eine schematische Darstellung einer Sensoreinheit gemäß der vorliegenden Erfindung; und
- Fig. 3: ein Ablaufdiagramm eines Verfahrens gemäß der vorliegenden Erfindung.

In der nachfolgenden Figurenbeschreibung soll das erfindungsgemäße Sensorsystem sowie das Verfahren gemäß der vorliegenden Erfindung anhand eines Einsatzes oder einer Verwendung in einem Hotelgebäude oder Ähnlichem erläutert werden. Andere Einsatzmöglichkeiten sind jedoch selbstredend möglich und bieten die gleichen, ähnliche oder gar noch weitere Vorteile als die nachfolgend beschriebenen Vorteile.

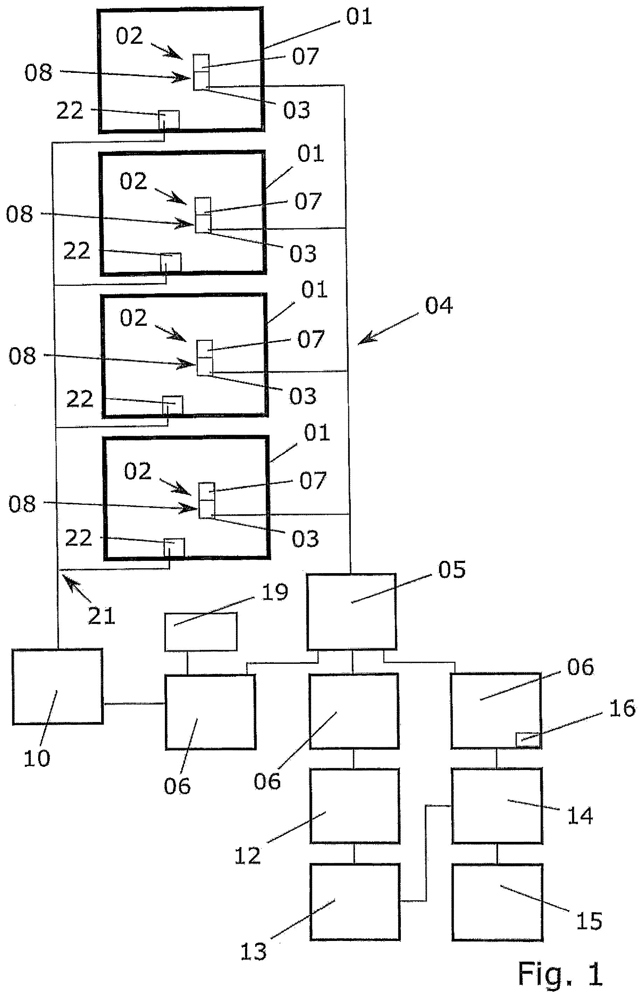

Die Fig. 1 zeigt ein Sensorsystem 20, gemäß der vorliegenden Erfindung. Das Sensorsystem 20 umfasst eine Vielzahl von Sensormodulen 02, welche jeweils einer Raumeinheit 01 zugeordnet sind. Im Beispiel der Fig. 4 sind insgesamt vier Raumeinheiten 01 dargestellt.

Die Raumeinheiten 01 sollen beispielsweise Hotelzimmer darstellen, wobei die Raumeinheiten 01 auf unterschiedlichen Stockwerken eines Hotelgebäudes angeordnet sein können. Der Einfachheit halber sind in der Fig. 1 lediglich vier Raumeinheiten 01 dargestellt. Das Sensorsystem lässt sich jedoch grundsätzlich auf eine unbegrenzte Anzahl von Raumeinheiten erweitern. Die in den Raumeinheiten 01, also in den Hotelzimmern, angeordneten Sensormodule 02 umfassen einerseits eine Sensoreinheit 03 zur Erfassung von Mikrobewegungen und darüber hinaus eine weitere Funktionseinheit 07. Im Beispiel der Fig. 1 soll es sich bei der Funktionseinheit 07 um einen Rauchmelder oder Rauchdetektor grundsätzlich bekannter Art handeln. Es ist jedoch vorgesehen, dass der Rauchmelder in Form der Funktionseinheit 07 mit der Sensoreinheit 03 des Sensormoduls 02 in einem gemeinsamen Gehäuse 08 angeordnet ist. Dadurch wird die Integration der Sensormodule in die bestehende Infrastruktur, also beispielsweise die Nachrüstbarkeit der Sensormodule 02 in die bestehende Hotelinfrastruktur erleichtert. Dazu kann besonders vorteilhaft vorgesehen sein, dass das Gehäuse 08 eine Form aufweist, die sich nach der Funktionseinheit 07, also nach dem Rauchmelder richtet.

In der Darstellung der Fig. 1 umfassen alle Sensormodule 02 der Raumeinheiten 01 eine Sensoreinheit sowie eine weitere Funktionseinheit 07. Dementsprechend sind auch alle Sensoreinheiten 03 mit einem Kommunikationsnetzwerk 04 verbunden, welches wiederum mit einer zentralen Recheneinheit 05 verbunden ist. Die Verbindung zwischen zentraler Recheneinheit 05 und Sensormodulen 02 oder Sensoreinheiten 03 kann kabelgebunden oder drahtlos erfolgen. Über das Kommunikationsnetzwerk 04 können besonders bevorzugt Sensorsignale der Sensoreinheiten 03 oder Informationen bzgl. gesendeter und/oder empfangener Sensorsignale der Sensoreinheiten 03 an die zentrale Recheneinheit übertragen werden. Zusätzlich kann jedoch auch vorgesehen sein, dass die Funktionseinheiten 07 das Kommunikationsnetzwerk 04 nutzen, um ebenfalls Daten oder Informationen, insbesondere mit der zentralen Recheneinheit oder mit an der zentralen Recheneinheit angeschlossenen Vorrichtungen auszutauschen.

Mittels der Sensoreinheiten 03 zur Erfassung von Mikrobewegungen, welche bevorzugt als UWB-Sensoren ausgebildet sind, können Mikrobewegungen in der Raumeinheit 01 erfasst werden. Mittels der Unterstützung der verbundenen zentralen Recheneinheit 05 ist es insbesondere möglich, durch die Erstellung einer Dopplerkarte und durch die Anwendung von Fourier-Transformationen Mikrodopplereffekte zu erkennen und zu verarbeiten. Dies wiederum ermöglicht die Erkennung von Grundschwingungen und harmonischen Oberschwingungen, mittels derer Mikrobewegungen eines Lebewesens, insbesondere eines Menschen zu detektieren, welche auf der Körperoberfläche durch den Herzschlag oder den Puls ausgelöst oder verursacht werden. Anhand derartiger charakteristischer Mikrobewegungen kann mit besonders hoher Sicherheit und Zuverlässigkeit auf die Anwesenheit oder die Abwesenheit eines Lebewesens, insbesondere eines Menschen in den jeweiligen Raumeinheiten 01 geschlossen werden.

Folglich ist mit dem erfindungsgemäßen Sensorsystem 20, insbesondere mittels der Sensoreinheiten 03 in Verbindung mit der zentralen Recheneinheit 05 die Anwesenheit oder die Abwesenheit eines Lebewesens, insbesondere eines Menschen, im Beispiel der Fig. 1 eines Hotelgastes, in der Raumeinheit 01, also in dem Hotelzimmer, bestimmbar.

Die Information über die Anwesenheit und die Abwesenheit von Hotelgästen in den Raumeinheiten 01, wie sie von den Sensoreinheiten 03 und der zentralen Recheneinheit 05 über die Mikrobewegungen in den Raumeinheiten 01 festegestellt wird, wird an die mit der zentralen Recheneinheit 05 verbundenen Anwendungseinheiten 06 übermittelt. Die Anwendungseinheiten 06 sind im Beispiel der Darstellung der Fig. 1 als drei einzelne separate Anwendungseinheiten 06 dargestellt. Alternativ könnten die Anwendungseinheiten 06 auch zu einer gemeinsamen Anwendungseinheit zusammengefasst werden. Ebenfalls könnte vorgesehen sein, die Anwendungseinheiten 06 in die zentrale Recheneinheit 05 zu integrieren. Die Anwendungseinheit 06 oder die Anwendungseinheiten 06 kann/können beispielsweise mit einer Speichereinheit 19 verbunden sein, in der anwendungsspezifische oder anwendungsbezogene Informationen und Parameter gespeichert sind. Dabei kann es sich einerseits um statische als auch um veränderliche Eigenschaften und Parameter handeln. Beispielsweise kann in der Speichereinheit 19 hinterlegt sein, an welchen Tagen des Jahres um jeweils wie viel Uhr ein Sonnenaufgang und/oder ein Sonnenuntergang stattfindet oder zu welchem Tageszeitpunkt ein gewisser Dämmerungswert vorliegt oder erwartet wird. Alternativ könnten in der Speichereinheit 19 auch Informationen sicherheitstechnischer Natur hinterlegt sein. Beispielsweise könnte hinterlegt sein, welche Raumeinheiten 01, also welche Hotelzimmer mit einem besonderen Rettungsmittel, beispielsweise einer Rettungsleiter erreichbar sind. Auch könnte in der Speichereinheit 19 hinterlegt sein, zu welchem Zeitpunkt oder an welchem Tag in der Vergangenheit zuletzt bestimmte Raumeinheitsarbeiten, beispielsweise Reinigungs- und/oder Hauskeeping-Arbeiten durchgeführt wurden. Neben einer Verbindung mit der Speichereinheit 19 kann die Anwendungseinheit 06 oder können die Anwendungseinheiten 06 jedoch auch mit weiteren Informationssystemen oder Messsystemen verknüpft sein, die die Anwendungseinheit 06 mit aktuellen oder situationsbezogenen Informationen über die Raumeinheiten 01 und/oder das Gebäude insgesamt versorgen. Beispielsweise kann die Anwendungseinheit 06 derart mit den Funktionseinheiten 07, also mit den Rauchmeldern verbunden sein, dass der Zustand des Rauchmelders der Funktionseinheit 07 der Anwendungseinheit 06 bekannt ist oder von der Anwendungseinheit 06 abgefragt werden kann. Auch weitere anwendungsspezifische dynamische oder aktuelle Informationen können von der Anwendungseinheit abgefragt oder der Anwendungseinheit 06 zur Verfügung gestellt werden, wozu entsprechend bestehende datentechnische Verbindungen und Schnittstellen genutzt oder eingerichtet werden können. Beispielsweise kann vorgesehen sein, dass die Anwendungseinheit 06 sowohl einen Istwert der Raumtemperatur der Raumeinheiten 01 zur Verfügung gestellt bekommt. Auch ein von einem Hotelgast, also einem ggf. in der Raumeinheit 01 anwesenden oder abwesenden Lebewesen eingestellter Temperatursollwert der Raumtemperatur der Raumeinheit 01 kann an die Anwendungseinheit 06 übermittelt werden.

Auch weitere Sensorsignale von in der Fig. 1 nicht dargestellten Sensoreinheiten, die beispielsweise eine optische oder räumliche Erfassung der Raumeinheit 01 ermöglichen, können berücksichtigt werden, beispielsweise um neben Anwesenheit auch die Lage, Bewegung und/oder Beschleunigung eines Lebewesens zu erfassen.

Jeweils abhängig von der entsprechenden Anwendungseinheit können aus den anwendungsspezifischen Informationen und Daten wie auch anhand der raumeinheitsbezogenen Informationen, insbesondere der Information der über die aktuelle Anwesenheit oder Anwesenheit von Lebewesen in den Raumeinheiten 01 als auch über die vergangene Abwesenheit und Anwesenheit sowie Abwesenheitsperioden und Anwesenheitsperioden von Lebewesen in den Raumeinheiten 01 von der Anwendungseinheit 06 jeweils ein anwendungsspezifisches und Ausgabesignal erzeugt werden. Beispielsweise kann das Ausgabesignal derart ausgestaltet sein, dass in einer Raumeinheit 01, in der eine Person oder ein Lebewesen über die entsprechenden Mikrobewegungen als aktuell anwesend detektiert wird eine eingestellte Solltemperatur der Raumtemperatur der Raumeinheit 01 von einer aktuellen Ist-Temperatur der Raumtemperatur der Raumeinheit 01 abweicht. Ein derartiges Ausgabesignal kann von der Anwendungseinheit 06 einer Steuereinheit 10 zur Verfügung gestellt werden, wobei die Steuereinheit 10 zur Steuerung zumindest einer Gebäudefunktion, insbesondere einer Raumeinheitsfunktion, beispielsweise einer Raumtemperatur einer Raumeinheit 01 eingerichtet ist, sodass die Steuereinheit 10 die Gebäudefunktion, insbesondere die Raumeinheitsfunktion in Abhängigkeit von dem Ausgabesignal der Anwendungseinheit 06 steuert. Im vorangehend skizzierten Beispiel kann also für das anwendungsspezifische Ausgabesignal der Anwendungseinheit 06, welches die Differenz einer Sollraumtemperatur und einer Ist-Raumtemperatur für eine Raumeinheit 01 betrifft, in der eine Person oder ein Lebewesen anwesend ist, die Steuereinheit 10 auf der Grundlage des Ausgabesignals der Anwendungseinheit 06 Steuersignale erzeugen, welche über das in der Fig. 1 skizzierte Steuernetzwerk 21 an in den Raumeinheiten 01 angeordnete Klimatisierungseinrichtungen übertragen oder weitergegeben werden, um die Differenz zwischen der Sollraumtemperatur und der Ist-Raumtemperatur für diejenigen Raumeinheiten 01 auszugleichen, in denen ein Lebewesen oder eine Person als anwesend detektiert wird. Ggf. kann auch die Anwendungseinheit 06 und die Steuereinheit 10 einer gemeinsamen Einheit realisiert werden.

Alternativ oder zusätzlich zu einer Steuereinheit 10 kann eine Anwendungseinheit 06 auch mit einer Organisationseinheit 12 verbunden sein. Die Organisationseinheit 12 soll beispielsweise Gebäudearbeiten, insbesondere Raumeinheitsarbeiten planen und koordinieren und damit den Betrieb des Gebäudes, in dem das Sensorsystem 20 installiert ist, verbessern oder effizienter durchführbar machen. Dementsprechend kann der Organisationseinheit 12 seitens der Anwendungseinheit 06 ein Ausgabesignal bereitgestellt werden, welches insbesondere die aktuelle Anwesenheit von Lebewesen in den Raumeinheiten 01 sowie die vergangenen Anwesenheiten und Abwesenheiten sowie Anwesenheitsphasen und Abwesenheitsphasen von Lebewesen in den Raumeinheiten 01 betrifft. Mit anderen Worten ausgedrückt, kann das Ausgabesignal der Anwendungseinheit 06, welche mit der Organisationseinheit 12 verbunden ist, vorrangig die Anwesenheit von Hotelgästen in den jeweiligen Hotelzimmern oder Raumeinheiten 01 und die Belegung der Hotelzimmer oder Raumeinheiten in Vergangenheit, insbesondere in einer vergangenen Nacht, wiederspiegeln. Aufbauend auf diesem Ausgabesignal der Anwendungseinheit 06 kann vorgesehen sein, dass die Organisationseinheit 12 einen, ggf. sich dynamisch aufgrund der Sensorsignale der Sensoreinheiten 03 sich verändernden Reinigungsplan oder Hauskeeping-Ablauf erstellt, um ein möglichst zeiteffektives und für die Hotelgäste angenehmes und komfortables Reinigen der Hotelzimmer, also der Raumeinheiten 01 zu ermöglichen. Dazu kann vorgesehen sein, dass die Organisationseinheit 12 wiederum mit einer Ausgabeeinheit 13 verbunden ist. Bei der Ausgabeeinheit 13 kann es sich beispielsweise um einen Drucker, einen Bildschirm, ein Smartphone oder dergleichen handeln. Die Ausgabeeinheit 13 dient dazu, die Planung der Raumeinheitsarbeiten den entsprechenden Arbeitskräften oder Hotelmitarbeiten anzuzeigen oder zu vermitteln, die Arbeiten durchführen sollen.

Zudem kann eine in der Fig. 1 nicht dargestellte Serviceeinheit vorgesehen sein, die einem in der Raumeinheit befindlichen Gast oder Lebewesen mögliche Serviceleistungen oder Servicedienste vorschlägt oder ausgibt, wobei die Servicedienste auf der Grundlage des Ausgangssignals der Anwendungseinheit 06 ermittelt werden. Beispielsweise kann einem Hotelgast ein Wellnessangebot, beispielsweise eine Massage innerhalb der Raumeinheit 01, also dem Hotelzimmer, oder in einer sonstigen Raumeinheit des Hotels unterbreitet werden. Es kann aber auch ein Unterhaltungsangebot, wie beispielsweise ein Sightseeing-Angebot unterbreitet werden. Dabei kann neben der Anwesenheit in der Raumeinheit 01 auch auf weitere statische oder dynamische Informationen zurückgegriffen werden, welche der Anwendungseinheit 06 übermittelt oder zur Verfügung gestellt werden. Beispielsweise können Buchungs- oder Anmeldeinformationen, die Präferenzen angeben oder durch die auf persönliche Vorlieben geschlossen werden kann, berücksichtigt werden, um ein Ausgangssignal der Anwendungseinheit 06 zu erzeugen, was den Bedürfnissen oder den Wünschen des Nutzers oder Hotelgasts entspricht.

Alternativ oder zusätzlich kann vorgesehen sein, dass eine Anwendungseinheit 06 mit einer Sicherheitseinheit 14 verbunden ist. Die Sicherheitseinheit 14 kann zum Erzeugen von Sicherheitseinheiten und/oder zum Koordinieren und/oder Unterstützen von Rettungsmaßnahmen und/oder zur Koordinierung von Wachschutz- oder Polizeieinsätzen eingerichtet sein. Anhand der Sensorsignale der Sensoreinheiten 03 und der Aufbereitung oder Auswertung zur Identifizierung der Anwesenheit oder Abwesenheit von Lebewesen in den Raumeinheiten 01 anhand der festgestellten Mikrobewegungen kann beispielsweise von der Anwendungseinheit 06 unter Berücksichtung von Informationen über die Zugänglichkeit der jeweiligen Raumeinheiten mittels entsprechenden Rettungsmitteln ein Ausgabesignal erzeugt werden, welche von der Sicherheitseinheit 14 weiterverarbeitet wird. Beispielsweise kann die Sicherheitseinheit 14 anhand des Ausgangssignals der Anwendungseinheit 06 dem Hotelpersonal über die Ausgabeeinheit 13 Sicherheitshinweise ausgeben, beispielsweise in welchen Hotelzimmern oder Raumeinheiten 01 ein Rauchmelder oder eine Funktionseinheit 07 aktiviert wurde und gleichzeitig ein Lebewesen als anwesend detektiert wurde. Alternativ kann jedoch auch vorgesehen sein, dass die Sicherheitseinheit 14 mit einer Leitstelle 15 von Einsatzkräften verbunden ist, und eine Kommunikation mit der Leitstelle 15 dahingehend erfolgt, in welchen Hotelzimmern oder Raumeinheiten 01 Hotelgäste anwesend sind und eine Evakuierung über spezielle Rettungsmittel, beispielsweise Rettungsleiter in einem Notfall benötigen. Außerdem kann vorgesehen sein, dass die Anwendungseinheit 06 ein Vorhersagemittel 16 umfasst, welches im Rahmen der anwendungsbezogenen und raumeinheitsbezogenen Verarbeitung der Sensorsignale eine Vorhersage über einen zukünftigen Zustand von zumindest einer Raumeinheit anfertigt. Beispielsweise kann mittels des Vorhersagemittels 16 vorhergesagt werden, ob in einem Notfall, beispielsweise einem Gebäudebrand aufgrund der Vitalfunktion eines in einer Raumeinheit befindlichen Hotelgast davon ausgegangen werden kann, dass der Hotelgast die Evakuierung selbstständig betreiben kann oder fremd evakuiert werden muss, da beispielsweise eine Bewusstlosigkeit bereits vorliegt oder vorhergesagt ist. In diesem Fall können über die Sicherheitseinheit 16 ebenfalls entsprechende Warnhinweise oder Koordinierungsmaßnahmen erzeugt und an die Ausgabeeinheit 13 oder die Leitstelle 15 übermittelt werden.

Alternativ oder zusätzlich können Mustererkennungsmittel zum Einsatz kommen, welche in der Darstellung der Fig. 1 nicht gezeigt sind. Die Mustererkennungsmittel können beispielsweise erkennen, ob im Falle eines Brandes oder eines sonstigen Notfalls ein in der Raumeinheit 01 befindlicher Hotelgast eine panische, nicht rationale oder unkoordiniert wirkende Reaktion zeigt, so dass die Hilfs- und/oder Rettungsmaßnahmen besonders auf den Hotelgast zugeschnitten werden können, um die Panik zu lindern oder zu überwinden.

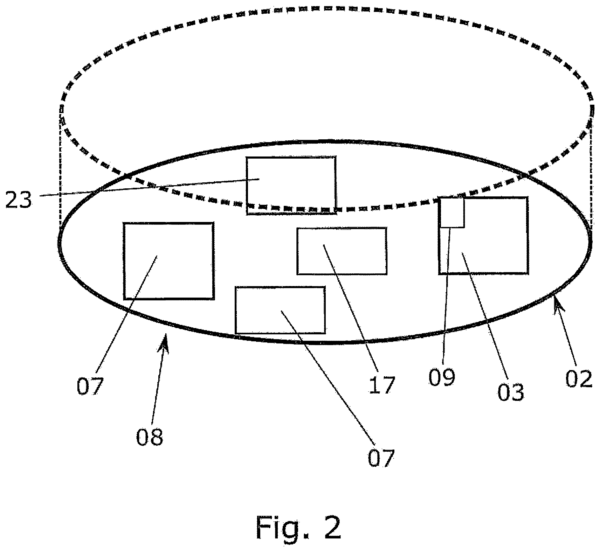

Fig. 2 zeigt eine schematische Darstellung eines Sensormoduls 02 umfassend eine Sensoreinheit 03. Neben der Sensoreinheit 03 umfasst das Sensormodul 02 eine weitere Funktionseinheit 07, beispielsweise einen Rauchsensor. Das Sensormodul 02 kann zudem eine Steuerelektronik 17 aufweisen. Außerdem kann das Sensormodul 02 eine Energieversorgungseinheit 18, insbesondere ein Akkumulator umfassen. Schließlich kann das Sensormodul 02 eine Kommunikationsschnittstelle 23 aufweisen, die beispielsweise zur drahtlosen Kommunikation eingerichtet ist. Durch die Kombination der Sensoreinheit 03 mit einer weiteren Funktionseinheit 07 kann das erfindungsgemäße Sensorsystem besonders platzsparend installiert werden. Außerdem kann das Sensorsystem besonders einfach und effektiv nachgerüstet werden, wenn die Sensoreinheit 03 mit einer weiteren Funktionseinheit 07, beispielsweise einem Rauchmelder in einem gemeinsamen Sensormodul 02 verknüpft wird. Ganz besonders vorteilhaft kann dazu vorgesehen sein, dass das in der Fig. 2 lediglich angedeutete gemeinsame Gehäuse 08 des Sensormoduls 02 eine Form aufweist, die sich nach zumindest der einen weiteren Funktionseinheit 07 des Sensormoduls 02 richtet. Die Sensoreinheit 03 umfasst bevorzugt einen Radarsensor 09, insbesondere einen kombinierten Radarsender und Empfänger, der beispielsweise als UWB-Transceiver ausgebildet sein kann. Dadurch wird ermöglicht, mittels der Sensoreinheit 03 Mikrobewegungen in einer Raumeinheit mit hoher räumlicher Auflösung zu erfassen.

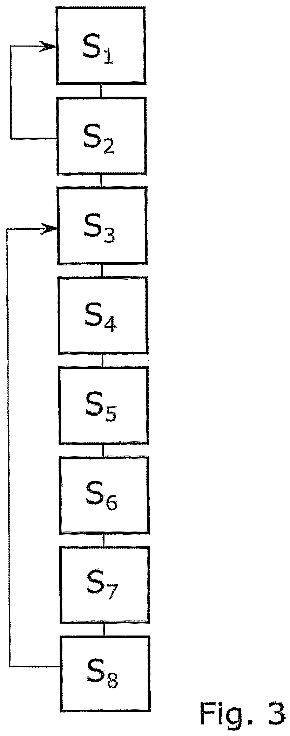

Die Fig. 3 zeigt ein schematisiertes Ablaufdiagramm des erfindungsgemäßen Verfahrens. In einem ersten Verfahrensschritt S₁ werden von einer Vielzahl von Sensormodulen, welche jeweils einer Raumeinheit zuordnet sind, ein Zustand der Raumeinheit erfasst. Insbesondere werden mit den Sensoreinheiten 03 Radarsignale ausgesendet und reflektierte Radarsignale empfangen. Die entsprechenden Sensorsignale oder Informationen über die Sensorsignale werden in einem zweiten Verfahrensschritt S₂ an eine zentrale Recheneinheit 05 übermittelt. Die Verfahrensschritte S₁ und S₂ können dabei dauerhaft wiederholt werden. Im Verfahrensschritt S₃ stellt eine Anwendungseinheit eine Informationsanfrage an die zentrale Recheneinheit 05, um Informationen über die Mikrobewegungen, insbesondere die Anwesenheit von Lebewesen, in den Raumeinheiten 01 zu empfangen. Im anschließenden Verfahrensschritt S₄ werden entsprechende Informationen über die Mikrobewegungen in den Raumeinheiten von der zentralen Recheneinheit 05 an die Anwendungseinheiten 06 gesendet und entsprechend von der Anwendungseinheit 06 empfangen. Im Verfahrensschritt S₅ wird von der Anwendungseinheit 06 neben den von der zentralen Recheneinheit 05 empfangenen Informationen über die Mikrobewegungen in den Raumeinheiten 05 auf weitere statische oder dynamische, insbesondere anwendungsbezogene oder raumeinheitsbezogene Informationen zugegriffen. Daran anschließend erstellt die Anwendungseinheit 06 im Verfahrensschritt S₆ ein anwendungsspezifisches und raumeinheitsbezogenes Ausgabesignal, welches dann in einem weiteren Verfahrensschritt S₇ an eine Steuereinheit 10, eine Organisationseinheit 12 oder eine Sicherheitseinheit 14 weitergegeben oder übermittelt wird. In einem abschließenden Verfahrensschritt S₈ kann dann von der Steuereinheit eine entsprechende Steuerung der Gebäudefunktion oder der Raumeinheitsfunktion veranlasst, eine Planung der Gebäudearbeiten, insbesondere der Raumeinheitsarbeiten durchgeführt und entsprechende Koordinierungsmaßnahmen von Einsatzkräften oder Ausgabe von Sicherheitshinweise durch die Sicherheitseinheit veranlasst werden. Nach Durchlaufen des Verfahrensschritts S₈ kann das Verfahren in den Verfahrensschritt S₃ zurückspringen, indem seitens der Anwendungseinheiten erneut Anfragen an die zentrale Recheneinheit 05 gesendet werden.

### Bezugszeichen

- 01: Raumeinheit
- 02: Sensormodul
- 03: Sensoreinheit
- 04: Kommunikationsmerkmal
- 05: Recheneinheit
- 06: Anwendungseinheit
- 07: Funktionseinheit
- 08: Gehäuse
- 09: Radarsensor
- 10: Steuereinheit
- 11: Klimatisierungseinrichtung
- 12: Organisationseinheit
- 13: Ausgabeeinheit
- 14: Sicherheitseinheit
- 15: Einsatzleistelle
- 16: Vorhersagemittel
- 17: Steuerelektronik
- 18: Energieversorgungseinheit
- 19: Speichereinheit
- 20: Sensorsystem
- 21: Steuernetzwerk
- 22: Gebäudefunktionseinheit
- 23: Kommunikationsschnittstelle

## Patentansprüche

1. Gebäude-Sensorsystem, für ein integratives Gebäudemanagement, umfassend
- eine Vielzahl von Sensormodulen (02), welche jeweils einer Raumeinheit (01) zugeordnet sind und einen Zustand der Raumeinheit (01) erfassen,
- zumindest eine zentrale Recheneinheit (05), umfassend zumindest eine Anwendungseinheit (06),
**dadurch gekennzeichnet, dass**
die Sensormodule (02) eine Sensoreinheit (03) zur Erfassung von Mikrobewegungen umfassen und jede Sensoreinheit (03) mit einem Kommunikationsnetzwerk (04) verbunden ist, über welches die Sensoreinheit (03) mit der zumindest einen zentralen Recheneinheit (05) kommuniziert, welche dazu eingerichtet ist, Mikrobewegungen in den Sensorsignalen der Sensoreinheiten (03) zu identifizieren,
die Sensormodule (02) einen Bewegungssensor und einen Rauch-Detektor aufweisen,
die zentrale Recheneinheit (05) mit der zumindest einen Anwendungseinheit (06) verbunden ist, welche dazu eingerichtet ist, Anfragen über Mikrobewegungen in den Raumeinheiten (01) an die zentrale Recheneinheit (05) zu senden, Informationen über die Mikrobewegungen in den Raumeinheiten (01) zu empfangen, die empfangenen Informationen über die Mikrobewegungen anwendungsbezogen und raumeinheitsbezogen zu verarbeiten und ein anwendungsspezifisches Ausgabesignal zu erzeugen, und
die Anwendungseinheit (06) ferner dazu eingerichtet ist, in dem Ausgabesignal eine Evakuierungs- oder Rettungsdringlichkeitsstufe für die jeweilige Raumeinheit (01) in Abhängigkeit der Anwesenheit oder Abwesenheit eines Lebewesens in der Raumeinheit (01), identifizierten Vitalfunktionen, nämlich Mikrobewegungen, hinsichtlich des Vitalzustands einer in der Raumeinheit (01) befindlichen Person und einer Rauchentwicklung in der jeweiligen Raumeinheit (01) festzulegen.

2. Gebäude-Sensorsystem nach Anspruch 1,
**dadurch gekennzeichnet,**
**dass** die Sensormodule (02) mit der Sensoreinheit (03) zumindest eine weitere Sensoreinheit (03) zur Erfassung der Lage und/oder Beschleunigung des Lebewesens aufweisen.

3. Gebäude-Sensorsystem nach einem der vorstehenden Ansprüche, **dadurch gekennzeichnet, dass** die Sensormodule (02) ein Gehäuse (08) umfassen, dessen Form sich nach dem Rauch-Detektor richtet, wobei der Rauch-Detektor mit der Sensoreinheit (03) des Sensormoduls (02) in dem gemeinsamen Gehäuse (08) angeordnet ist.

4. Gebäude-Sensorsystem nach einem der vorstehenden Ansprüche, **dadurch gekennzeichnet, dass** die Sensoreinheit (03) einen Radarsensor, insbesondere einen Ultra-Wide-Band (UWB) Sensor umfasst.

5. Gebäude-Sensorsystem nach einem der vorstehenden Ansprüche, **dadurch gekennzeichnet, dass** die Recheneinheit (05) und/oder die Sensoreinheiten (03) dazu eingerichtet sind, eine Mikrobewegung einer Vitalfunktion eines Lebewesens in Kombination mit einer Mikrobewegung des Lebewesens in einer Raumeinheit (01) in den Sensorsignalen zu identifizieren.

6. Gebäude Sensorsystem nach einem der vorstehenden Ansprüche, **dadurch gekennzeichnet, dass** die Recheneinheit (05) und/oder die Sensoreinheiten (03) dazu eingerichtet sind, eine Atemfrequenz und/oder eine Frequenz eines Herzschlages eines Lebewesens in Kombination mit einer Mikrobewegung des Lebewesens in einer Raumeinheit (01) in den Sensorsignalen zu identifizieren.

7. Gebäude-Sensorsystem nach einem der vorstehenden Ansprüche, **gekennzeichnet durch** eine Steuereinheit (10) zur Steuerung zumindest einer Gebäudefunktion, insbesondere einer Raumeinheitsfunktion, wobei zumindest eine Anwendungseinheit (06) mit der Steuereinheit (10) verbunden ist und die Steuereinheit (10) dazu eingerichtet ist, die Gebäudefunktion, insbesondere die Raumeinheitsfunktion, in Abhängigkeit von dem Ausgabesignal der Anwendungseinheit (06) zu steuern.

8. Gebäude-Sensorsystem nach einem der vorstehenden Ansprüche, **gekennzeichnet durch** zumindest eine Organisationseinheit (12), welche zur Planung von Raumeinheitsarbeiten, insbesondere Wartungs- und Pflegearbeiten, eingerichtet ist, wobei eine Anwendungseinheit (06) mit der Organisationseinheit (12) verbunden ist und die Organisationseinheit (12) dazu eingerichtet ist die Planung von Gebäudearbeiten in Abhängigkeit von dem Ausgabesignal der Anwendungseinheit (06) durchzuführen.

9. Gebäude-Sensorsystem nach einem der vorstehenden Ansprüche, **gekennzeichnet durch** eine Sicherheitseinheit (14), zum Erzeugen von Sicherheitshinweisen und/oder zum Koordinieren oder Unterstützen von Rettungsmaßnahmen und/oder zur Koordinierung von Wachschutz- oder Polizeieinsätzen eingerichtet ist, wobei zumindest eine Anwendungseinheit (06) mit der Sicherheitseinheit (14) verbunden ist und die Sicherheitseinheit (14) dazu eingerichtet ist in Abhängigkeit von dem Ausgabesignal der Anwendungseinheit (06) die Sicherheitshinweise zu erzeugen und/oder die Rettungsmaßnahmen und/oder Wachschutz- oder Polizeieinsätze zu koordinieren oder zu unterstützen.

10. Beherbergungseinrichtung, insbesondere Hotel, Schiff oder Kreuzfahrtschiff, umfassend ein Gebäude-Sensorsystem nach einem der Ansprüche 1 bis 9, wobei die Raumeinheiten (01) zumindest ein Hotelzimmer umfassen.

11. Medizinische Unterbringungseinrichtung, insbesondere Krankenhaus und/oder Alters- und/oder Pflegeheim, umfassend ein Gebäude-Sensorsystem nach einem der Ansprüche 1 bis 9, wobei die Raumeinheiten (01) zumindest einen Patienten- oder Bewohnerlagerungsbereich, insbesondere ein Patienten- und/oder Bewohnerbett, umfassen.

12. Verfahren für ein integratives Gebäudemanagement mittels eines Gebäude-Sensorsystems nach einem der Ansprüche 1 bis 9.

## Claims

1. A building-sensor-system, for integrative building management, including
- a plurality of sensor modules (02), which are associated to a room unit (01), respectively, and which registrate a state of the room unit (01),
- at least one central computing unit (05), including at least one application unit (06)
**characterized in that**
the sensor module (02) includes a sensor unit (03) for the registration of micromovements and each sensor unit (03) is connected to a communication network (04) over which the sensors unit communicates with the at least one central computation unit (05) which is adapted to identify micromovements in the sensor signals of the sensor units (03),
the sensor module (02) includes a motion sensor and a smoke detector,
the central computing unit (05) is connected with the at least one application unit (06) which is adapted to send inquiries regarding the micromovements in the room units (01) to the central computing units (05), to receive information regarding the micromovements in the room units (01), to process the received information regarding the micromovements in an application- and room-unit-sensitive fashion and to generate an application-specific output signal, and
the application unit (06) is further adapted to define within the output signal an evacuation- or rescue-urgency-level for the respective room unit (01), depending on the presence or absence of a living being in the room unit (01), identified vital functions, namely micromovements, with regard to the vital condition of a person present in the room unit (01) and the development of smoke.

2. The building-sensor-system of claim 1, **characterized in that** the sensor modules (02) with the sensor unit (03) includes at least one more sensor unit (03) for determining the position and/or acceleration of a living being.

3. The building-management-system of any previous claim, **characterized in that** the sensor modules (02) include a housing (08), the shape of which conforms to the smoke detector, whereby the smoke detector is arranged together with the sensor unit (03) of the sensor module (02) within the shared housing (08).

4. The building-sensor-system of any previous claim, **characterized in that** the sensor unit (03) includes a radar sensor, particularly an ultra-wide-band (UWB) sensor.

5. The building-sensor-system of any previous claim, **characterized in that** the computing unit (05) and/or the sensor units (03) are adapted to identify, within the sensor signals, a micromovement of a vital function of a living being in combination with a micromovement of the living being in a room unit (01).

6. The building-sensor-system of any previous claim, **characterized in that** the computing unit (05) and/or the sensor units (03) are adapted to identify, within in the sensor signals, a breathing frequency and/or a frequency of a heartbeat of a living being in combination with a micromovement of the living being in a room unit (01).

7. The building-sensor-system of any previous claim, **characterized by** a control unit (10) for controlling at least one building function, particularly a room unit function, whereby at least one application unit (06) is connected to the control unit (10) and the control unit (10) is adapted to control the building function, particularly the room unit function, depending on the output signal of the application unit (06).

8. The building-sensor-system of any previous claim, **characterized by** at least one organisation unit (12) which is adapted to schedule room unit work, particularly maintenance and servicing work, wherein the application unit (06) is connected to organisation unit (12) and the organisation unit (12) is adapted to perform the scheduling of building work, depending on the output signal of the application unit (06).

9. The building-sensor-system of any previous claim, **characterized by** a security unit (14) which is adapted for generating security notifications and/or for coordinating or supporting rescue measures and/or security or police operations, wherein at least one application unit (06) is connected to the security unit (14) and the security unit (14) is adapted to, depending on the output signal of the application unit (06), generate the security notifications and/or coordinate or support the rescue measures and or security or police operations.

10. An accommodation facility, particularly a hotel, ship or cruise ship, including a building-sensor-system of any claim of the claims 1-9, whereby the room units (01) include at least one hotel room.

11. A medical accommodation facility, particularly a hospital, retirement and/or nursing home, including a building-sensor-system of any claim of the claims 1-9, whereby the room units (01) include at least one patient or resident positioning region, particularly a patient and/or resident bed.

12. A method for an integrative building management by means of the building-sensor-system of any claim of the claims 1-9.

## Revendications

1. Système de capteurs de bâtiment pour une gestion intégrée des bâtiments, comprenant
- une pluralité de modules de capteurs (02) qui sont chacun associés à une unité spatiale (01) et détectent un état de l'unité spatiale (01),
- au moins une unité centrale de calcul (05) comprenant au moins une unité d'application (06),
**caractérisé en ce que**
les modules de capteur (02) comprennent une unité de capteur (03) pour détecter des micro-mouvements et chaque unité de capteur (03) est reliée à un réseau de communication (04) par l'intermédiaire duquel l'unité de capteur (03) communique avec la ou les unités centrales de calcul (05) qui sont conçues pour identifier des micro-mouvements dans les signaux de capteur des unités de capteur (03),
les modules de capteur (02) comportent un capteur de mouvement et un détecteur de fumée,
l'unité centrale de calcul (05) est reliée à au moins une unité d'application (06) qui est conçue pour envoyer à l'unité centrale de calcul (05) des demandes concernant les micro-mouvements dans les unités spatiales (01), de recevoir des informations sur les micro-mouvements dans les unités spatiales (01), de traiter les informations reçues sur les micro-mouvements en fonction de l'application et de l'unité spatiale et de générer un signal de sortie spécifique à l'application, et
l'unité d'application (06) étant en outre conçue pour définir, dans le signal de sortie, un niveau d'urgence d'évacuation ou de sauvetage pour l'unité spatiale (01) correspondante en fonction de la présence ou de l'absence d'un être vivant dans l'unité spatiale (01), des fonctions vitales identifiées, à savoir des micro-mouvements, en ce qui concerne l'état vital d'une personne se trouvant dans l'unité spatiale (01) et d'un dégagement de fumée dans l'unité spatiale correspondante (01).

2. Système de capteurs de bâtiment selon la revendication 1, **caractérisé en ce que**
les modules de capteurs (02) comportent, avec l'unité de capteurs (03), au moins une autre unité de capteurs (03) pour détecter la position et/ou l'accélération de l'être vivant.

3. Système de capteurs de bâtiment selon l'une des revendications précédentes, **caractérisé en ce que** les modules de capteurs (02) comprennent un boîtier (08) dont la forme est adaptée au détecteur de fumée, le détecteur de fumée étant disposé avec l'unité de capteur (03) du module de capteur (02) dans le boîtier commun (08).

4. Système de capteurs de bâtiment selon l'une des revendications précédentes, **caractérisé en ce que** l'unité de capteur (03) comprend un capteur radar, en particulier un capteur à bande ultra large (UWB).

5. Système de capteurs de bâtiment selon l'une des revendications précédentes, **caractérisé en ce que** l'unité de calcul (05) et/ou les unités de capteurs (03) sont conçues pour identifier, dans les signaux des capteurs, un micro-mouvement d'une fonction vitale d'un être vivant en combinaison avec un micro-mouvement de l'être vivant dans une unité spatiale (01).

6. Système de capteurs de bâtiment selon l'une des revendications précédentes, **caractérisé en ce que** l'unité de calcul (05) et/ou les unités de capteurs (03) sont conçues pour identifier, dans les signaux des capteurs, une fréquence respiratoire et/ou une fréquence cardiaque d'un être vivant en combinaison avec un micro-mouvement de l'être vivant dans une unité spatiale (01).

7. Système de capteurs de bâtiment selon l'une des revendications précédentes, **caractérisé par** une unité de commande (10) permettant de commander au moins une fonction du bâtiment, en particulier une fonction d'unité spatiale, au moins une unité d'application (06) étant reliée à l'unité de commande (10) et l'unité de commande (10) étant conçue pour commander la fonction du bâtiment, en particulier la fonction d'unité spatiale, en fonction du signal de sortie de l'unité d'application (06).

8. Système de capteurs de bâtiment selon l'une des revendications précédentes, **caractérisé par** au moins une unité d'organisation (12) qui est configurée pour planifier des travaux d'unité spatiale, en particulier des travaux d'entretien et de maintenance, une unité d'application (06) étant reliée à l'unité d'organisation (12) et l'unité d'organisation (12) étant configurée pour réaliser la planification des travaux dans le bâtiment en fonction du signal de sortie de l'unité d'application (06).

9. Système de capteurs de bâtiment selon l'une des revendications précédentes, **caractérisé par** une unité de sécurité (14) conçue pour générer des consignes de sécurité et/ou pour coordonner ou assister des mesures de sauvetage et/ou pour coordonner des interventions de sécurité ou de police, au moins une unité d'application (06) étant reliée à l'unité de sécurité (14) et l'unité de sécurité (14) étant conçue pour générer les consignes de sécurité et/ou coordonner ou assister les mesures de sauvetage et/ou les interventions de sécurité ou de police en fonction du signal de sortie de l'unité d'application (06).

10. Établissement d'hébergement, en particulier hôtel, bateau ou bateau de croisière, comprenant un système de capteurs de bâtiment selon l'une des revendications 1 à 9, les unités spatiales (01) comprenant au moins une chambre d'hôtel.

11. Établissement médical, en particulier hôpital et/ou maison de retraite et/ou établissement médico-social, comprenant un système de capteurs de bâtiment selon l'une des revendications 1 à 9,
les unités spatiales (01) comprenant au moins une zone de couchage pour patients ou résidents, en particulier un lit pour patients et/ou résidents.

12. Procédé pour une gestion intégrée des bâtiments à l'aide d'un système de capteurs de bâtiment selon l'une des revendications 1 à 9.
